# EUROPÄISCHE PATENTSCHRIFT

(11) **EP 4 452 927 B1**
(45) Veröffentlichungstag und Bekanntmachung des Hinweises auf die Patenterteilung: **14.10.2026**
(21) Anmeldenummer: 22839723.8
(22) Anmeldetag: 16.12.2022
(51) Int. Cl.: C07C 227/42, C07C 229/56, C07C 229/60, C12P 13/00

(54) **VERFAHREN ZUR GEWINNUNG VON AMINOBENZOESÄURE AUS EINER WÄSSRIGEN MUTTERLAUGE**
METHOD FOR THE RECOVERY OF AMINOBENZOIC ACID FROM AN AQUEOUS MOTHER LIQUOR
PROCÉDÉ DE RÉCUPÉRATION DE L'ACIDE AMINOBENZOÏQUE À PARTIR D'UNE LIQUEUR MÈRE AQUEUSE

(30) Priorität: 20.12.2021 EP 21215923
(43) Veröffentlichungstag der Anmeldung: 30.10.2024
(73) Patentinhaber: Covestro Deutschland AG, 51373 Leverkusen (DE)
(72) Erfinder: KRAUSE, Jonas, 40764 Langenfeld (DE); FRANCISCO CASAL, Maria, 51375 Leverkusen (DE); KLOECKNER, Wolf, 51065 Köln (DE); BEDNARZ, Andreas, 53909 Zülpich (DE)
(74) Vertreter: Levpat
(86) Internationale Anmeldenummer: PCT/EP2022/086346
(87) Internationale Veröffentlichungsnummer: WO 2023/117756

(56) Entgegenhaltungen:
- WO-A1-2007/088346
- WO-A1-2015/124687

## Beschreibung

Die Arbeiten, die zu der vorliegenden Erfindung geführt haben, wurden vom deutschen Bundesministerium für Ernährung und Landwirtschaft unter dem Förderkennzeichen 22019918 finanziell gefördert.

Die vorliegende Erfindung betrifft ein Verfahren zur Gewinnung von Aminobenzoesäure aus einer wässrigen Mutterlauge, welche bei einer Kristallisation von Aminobenzoesäure anfällt. Das Verfahren umfasst einen **Schritt (A)** des Fermentierens eines geeigneten fermentierbaren Rohstoffes in Gegenwart von Mikroorganismen unter Bildung von Aminobenzoat-Anionen (H₂NC₅H₄COO⁻) und/oder Aminobenzoesäure (H₂NC₅H₄COOH bzw. H₃N⁺C₆H₄COO⁻), einen **Schritt (B)** des Kristallisierens von Aminobenzoesäure bei einem pH-Wert von 3,0 bis 4,7, wobei diese Kristallisation während und/oder nach der Fermentation durchgeführt werden kann, einen **Schritt (C)** des Extrahierens der wässrigen Mutterlauge der Kristallisation mit einem Alkanol mit 8 bis 12 Kohlenstoffatomen unter Erhalt einer ersten alkoholischen Phase enthaltend Aminobenzoesäure und einer ersten wässrigen Phase, einen **Schritt (D)** der Rückextraktion von Aminobenzoesäure aus der ersten alkoholischen Phase mit einer wässrigen Basen- oder Säurelösung unter Erhalt einer zweiten wässrigen Phase enthaltend Anionen (H₂NC₅H₄COO⁻) bzw. Kationen (H₃N⁺C₆H₄COOH) der Aminobenzoesäure und einer zweiten alkoholischen Phase, und einen **Schritt (E)** des Kristallisierens von Aminobenzoesäure aus der zweiten wässrigen Phase durch Rückführen dieser in die Kristallisation aus Schritt (B) oder in einer separaten Kristallisation.

Die Herstellung organischer Säuren durch fermentative Prozesse hat in der jüngeren Vergangenheit besondere Aufmerksamkeit erfahren. Unter den organischen Säuren, die fermentativ zugänglich sind, ist auch Aminobenzoesäure als ein wirtschaftlich bedeutsames Produkt hervorzuheben. Aminobenzoesäure findet bspw. Anwendung in der Herstellung von Farbstoffen, Geruchsstoffen oder Pharmazeutika. Ein weiteres Beispiel für eine Anwendung von Aminobenzoesäure ist ihre Verwendung in der Herstellung von Anilin durch Decarboxylierung. Anilin seinerseits ist insbesondere als Intermediat in der Herstellung von Isocyanaten von Bedeutung.

Die fermentative Herstellung von Aminobenzoesäure ist in der Literatur beschrieben. Beispielhaft sei auf Balderas-Hemandez, V. E. et al., "Metabolic engineering for improving anthranilate synthesis from glucose in Escherichia coli", Microb. Cell. Fact. 2009, 8, 19 (doi: 10.118611475-2859-8-19) verwiesen. Auch in der Patentliteratur finden sich hierzu Veröffentlichungen; siehe beispielsweise die internationale Patentanmeldung WO 2015/124687 A1, welche die zweistufige Herstellung von Anilin über *ortho-*Aminobenzoesäure als Zwischenprodukt betrifft, sowie die darin zitierte Literatur. Fermentationsprozesse laufen in einer wässrigen Umgebung ab und liefern im Allgemeinen im Falle der Herstellung von Aminobenzoesäure wässrige Produktgemische *(Fermentationsbrühen)* mit einem Massengehalt an Aminobenzoesäure im Bereich von 10,0 g/L bis 100 g/L.

Besondere Bedeutung hat das ortho-Isomer der Aminobenzoesäure, die *Anthranilsäure.* Im Stoffwechsel von Bakterien und Hefen wird Anthranilsäure im Shikiminsäureweg als natürliches Intermediat bei der Synthese von Tryptophan gebildet. Bei der biotechnologischen Produktion von Anthranilsäure wird deren Umsetzung im Stoffwechselweg reduziert oder unterbunden, um eine Akkumulation im Fermentationsmedium zu erzielen. Ein solches Konzept zur biotechnologischen Produktion von Anthranilsäure und ihrer anschließenden katalytischen Umsetzung zu Anilin ist in den bereits erwähnten internationalen Patentanmeldungen WO 2015/124686 A1 und WO 2015/124687 A1 beschrieben. Als möglicher rekombinanter Mikroorganismus wird die Verwendung von Bakterien aus der Familie der Corynebakterien oder Pseudomonaden beschrieben. Eine jüngere Anmeldung (WO 2017/102853 A1) beschreibt den Einsatz von Hefen.

Aber auch para-Aminobenzoesäure ist von Interesse. Die Synthese von *para-*Aminobenzoesäure kann im Stoffwechsel von Bakterien und Hefen über das Intermediat Chorismat erfolgen, das als Zwischenprodukt im Shikiminsäureweg entsteht. Chorismat wird enzymatisch zunächst zu 4-Amino-4-deoxychorismat und dann durch eine zweite Enzymreaktion zu para-Aminobenzoesäure umgesetzt. Ein Konzept zur biotechnologischen Produktion von Anilin über das Intermediat para-Aminobenzoesäure ist in der internationalen Anmeldung WO2014171205 beschrieben. Als möglicher rekombinanter Mikroorganismus wird auch in hier u.a. die Verwendung von Bakterien aus der Familie der Corynebakterien beschrieben.

Bei der fermentativen Herstellung von Aminobenzoesäure fällt ein stark verdünnter wässriger Produktstrom an. Das Wertprodukt Aminobenzoesäure liegt bei einer Fermentation im Bereich von pH 6 bis pH 9 (üblicher pH-Bereich bei der Verwendung von Bakterien als Mikroorganismen) überwiegend geladen als Aminobenzoesäure-Anion vor. Nach der Durchführung von für Fermentationen üblichen Aufarbeitungsschritten kann durch Einstellung des pH-Wertes auf einen Wert in der Nähe des oder am isoelektrischen Punkt das Wertprodukt Aminobenzoesäure in elektroneutraler Form als Feststoff ausgefällt werden. Die Aminobenzoesäure kann dann beispielsweise durch Filtration abgetrennt werden. Das abfiltrierte Produkt fällt zunächst in einem stark wasserhaltigen Zustand an *("slurry").* Dieses Produkt wird dann beispielsweise gewaschen und erforderlichenfalls (abhängig von der geplanten Verwendung) getrocknet oder auch in einem Lösungsmittel wie Anilin oder 1-Dodecanol aufgenommen (siehe WO 2015/124687 A1).

Bei der Filtration bleibt eine Mutterlauge zurück, die noch eine signifikante (der Löslichkeit von Aminobenzoesäure unter den jeweiligen Bedingungen entsprechende) RestKonzentration an Aminobenzoesäure enthält und daher vor ihrer Entsorgung als Abwasser von dieser gelösten Aminobenzoesäure möglichst quantitativ befreit werden sollte.

Die bereits mehrfach erwähnte internationale Anmeldung WO 2015/124687 A1 offenbart hierzu eine bevorzugte Ausführungsform, in welcher die bei der Kristallisation erhaltene Mutterlauge aufgearbeitet wird, um weitere Aminobenzoesäure (hier Anthranilsäure) zu gewinnen. Dies geschieht durch eine Sequenz aus einem Adsorptions- und einem Desorptionsschritt. Das erhaltene an Aminobenzoesäure angereicherte Desorbat wird in die Kristallisation zurückgeführt. Auf diese Weise werden Ausbeuteverluste verringert. Die Adsorption erfolgt dabei an Zeolithen oder Aktivkohle, die Desorption mit Wasser eines pH-Werts im Bereich von 5 bis 10 oder alternativ mit organischen Lösungsmitteln, insbesondere 1-Dodecanol. Eine Variante einer solchen Aufarbeitung durch eine Folge aus einem Adsorption- und Desorptionsschritt ist in der internationalen Anmeldung WO 2018/114841 A1 beschrieben. Das dort offenbarte Verfahren ist insbesondere dadurch gekennzeichnet, dass die Desorption in saurer Umgebung (pH -0,8 bis 3,0) durchgeführt wird. Als ein geeignetes Adsorptionsmittel wird Aktivkohle offenbart. Selbstverständlich eignen sich neben Aktivkohle noch weitere Adsorptionsmittel für das in WO 2018/114841 A1 beschriebene Verfahren. Insbesondere sind hier die in der Fachwelt geläufigen polymeren Adsorptionsmittel, vorzugsweise solche auf Polystyrol- und/oder Polydivinylbenzol-Basis, zu nennen. Typische Porengrößen bewegen sich im Bereich von 1,5 bis 65 nm, z. B. 4,5 bis 10 nm. Es gibt auch Adsorbertypen mit Mikro- und Makroporen. Kommerziell erhältliche Produkte sind beispielsweise unter den Markennamen Lewatit (z. B. OC 1064 MD PH oder AF 5), Macronet (z. B. MN 270, MN 202, MN 100 oder MN 102), PuroSorb (z. B. PAD600), AmberSorb (z. B. L493 oder 560) und Amberlite (z. B. XAD4) zu finden. Die Verwendung dieser (und anderer Adsorptionsmittel wie beispielsweise Aktivkohle) ist selbstverständlich nicht auf die in WO 2018/114841 A1 beschriebene saure Desorption beschränkt, vielmehr eignen sich diese auch für eine basische Desorption. Erfolgt die Desorption im Sauren, so empfiehlt es sich, von Zeit zu Zeit (beispielsweise nach fünf durchgeführten sauren Desorptionen) eine zusätzliche Regeneration des Adsorptionsmittels mit Base durchzuführen. Auf diese Weise wird das Adsorberbett von organischen Verunreinigungen befreit.

Verfahren unter Anwendung einer Abfolge von Adsorptions- und Desorptionsschritten haben den generellen Nachteil, dass bei Produktion in großtechnischem Maßstab sehr große Volumina an Adsorberbetten benötigt werden. Außerdem sind die zur Adsorption verwendeten Materialien nicht unbegrenzt regenerierbar, müssen also mindestens in Teilen regelmäßig ersetzt werden, was Kosten und Feststoffabfall zur Folge hat. Ein Verfahren umfassend die *Extraktion* der *Mutterlauge* zur Rückgewinnung darin gelöster Aminobenzoesäure wird von keiner der beiden genannten Anmeldungen offenbart.

Die internationale Patentanmeldung WO 2007/088346 A1 beschreibt ein Verfahren zur Gewinnung von Anthranilsäure aus der Mutterlauge einer Kristallisation zur Isolierung von Anthranilsäure, die durch eine Hofmann-Umlagerung erhalten wurde. Das Natriumcarbonat-haltige Produkt der Hofmann-Umlagerung wird mit Schwefelsäure auf einen pH-Wert von 4,2 eingestellt (a) und ausgefallene Anthranilsäure abfiltriert (b). Die zurückbleibende Mutterlauge wird bei einem pH-Wert von 4,2 mit einem organischen Lösungsmittel extrahiert, wobei Essigsäureester (insbesondere Ethyl- oder Butylacetat), Ketone (insbesondere 2-Butanon) und aromatische Kohlenwasserstoffe (insbesondere Toluol) als geeignete organische Lösungsmittel offenbart werden. Der nach Phasentrennung (c) anfallende organische Extrakt wird anschließend mit Natronlauge rückextrahiert (d), wobei Anthranilsäure als Anthranilat-Anion in die nach Phasentrennung (e) erhaltene wässrige Phase übergeht. Diese wässrige, an Anthranilsäure angereicherte Phase der Rückextraktion wird mit dem Produkt der Hofmann-Umlagerung vereint und gemeinsam mit diesem der Kristallisation zugeführt. Die in der Extraktion anfallende wässrige, an Anthranilsäure abgereicherte Phase wird mit Schwefelsäure auf pH 1,5 eingestellt, mit weiterem organischen Lösungsmittel versetzt (f) und in eine wässrige und organische Phase getrennt (g). Auf diese Weise gelangen organische Verunreinigungen in die organische Phase. Diese organische Phase wird mit der organischen Phase der Rückextraktion vereint und destilliert. Das Destillat kann als Lösungsmittel in den Prozess rezykliert werden. Der Destillationsrückstand wird verbrannt. Die wässrige Phase aus (g) wird nach Reinigung durch eine Fenton-Reaktion dem Abwasser zugeführt. Nachteilig an diesem Verfahren ist insbesondere die relativ hohe Wasserlöslichkeit der Essigsäureester und Ketone. Essigsäureester und Ketone lösen zwar Anthranilsäure gut, gelangen aber wegen der vergleichsweise hohen Wasserlöslichkeit in nicht vernachlässigbaren Anteilen in die wässrige Phase. Dies hat einerseits Ausbeuteverluste zur Folge und erhöht andererseits den Aufwand bei der Abwasserreinigung. Die vergleichsweise hohe Wasserlöslichkeit der Essigsäureester und Ketone macht eine Anwendung des in WO 2007/088346 A1 beschriebenen Extraktionsprozesses in der Gewinnung *fermentativ* hergestellter Anthranilsäure besonders problematisch, weil dort im Vergleich zu einem auf petrochemischen Rohstoffen basierendem Herstellverfahren die anfallenden Wasserströme und dadurch die absoluten Ausbeuteverluste sowie der Aufwand bei der Abwasserreinigung erheblich größer sind. Das in WO 2007/088346 A1 ebenfalls als geeignetes Lösungsmittel offenbarte Toluol weist die beschriebenen Nachteile zwar nicht auf, löst aber Anthranilsäure erheblich schlechter, was es schwierig macht, einen praktikablen Prozess im großtechnischen Maßstab mit Toluol als Extraktionsmittel zu entwickeln.

CN 104 016 871 A beschreibt die Extraktion von Anthranilsäure-haltigem Abwasser aus der Produktion von Anthranilsäuremethylester mit einer Mischung aus Tributylphosphat und Kerosin. Die Notwendigkeit des Einsatzes von Tributylphosphat führt zu einer unerwünschten Kostensteigerung.

CN 104 926 673 A beschreibt die Extraktion von para-Aminobenzoesäure-haltigen wässrigen Lösungen mit ionischen Flüssigkeiten. Ionische Flüssigkeiten sind teuer, sodass sie für eine wirtschaftliche Anwendung im großtechnischen Maßstab unbedingt rezykliert werden müssen. Im Gegensatz zur niedermolekularen organischen Lösungsmitteln als Extraktionsmittel können sie jedoch nicht einfach durch eine Destillation gereinigt werden, was eine Rezyklierung erschwert.

CN 109 850 976 A beschreibt die Extraktion von para-Aminobenzoesäure-haltigen wässrigen Lösungen mit einer Mischung aus Tributylphoshpat, 1-Octanol und Kerosin. Der Einsatz eines so komplexen Extraktionsmittels erhöht die Kosten und erschwert dessen Rezyklierung.

Die internationale Patentanmeldung WO 2015/124686 A1 beschreibt die Extraktion von *Anilin,* das durch Decarboxylierung einer Ammonium-Anthranilat-Lösung erhalten wurde, mit 1-Dodecanol. Die Anthranilsäure kann fermentativ oder petrochemisch hergestellt werden.

Keines der zuvor beschriebenen Extraktionsverfahren ist daher für die Rückgewinnung von Aminobenzoesäure aus einer Mutterlauge, die in der Herstellung von Aminobenzoesäure auf fermentativem (= biotechnologischem) Weg unter Isolierung der gebildeten Aminobenzoesäure durch Kristallisation anfällt, wirklich zufriedenstellend. Dies gilt insbesondere bei einer Produktion im großtechnischen Maßstab.

Es bestand also ein Bedarf an weiteren Verbesserungen auf dem Gebiet der Herstellung von Aminobenzoesäure durch Fermentationsprozesse umfassend einen Kristallisationsschritt. Insbesondere wäre es wünschenswert, die Aminobenzoesäure möglichst vollständig isolieren zu können. Hierzu ist es unerlässlich, den in der Mutterlauge der Kristallisation verbleibenden gelösten Anteil an Aminobenzoesäure zumindest in großen Anteilen aus dieser zurückzugewinnen. Insbesondere wäre es ferner wünschenswert, die Mutterlauge im Zuge einer solchen Aufarbeitung an organischen Verunreinigungen abzureichern, um die Aufbereitung bzw. Entsorgung dieses (beträchtlichen) wässrigen Stromes zu vereinfachen. In diesem Zusammenhang ist auch eine möglichst geringe Wasserlöslichkeit des Extraktionsmittels selbst von Bedeutung. Schließlich wäre es insbesondere wünschenswert, derartige aus der Mutterlauge entfernte organische Verunreinigungen auf effiziente Weise aus dem Prozess ausschleusen zu können.

Diesem Bedarf Rechnung tragend ist ein Gegenstand der vorliegenden Erfindung ein **Verfahren zur Gewinnung von Aminobenzoesäure aus einer wässrigen Mutterlauge enthaltend** gelöste **Aminobenzoesäure,** welche bei einer Kristallisation von Aminobenzoesäure anfällt, wobei das Verfahren folgende Schritte umfasst:
(A) Fermentieren eines Rohstoffes enthaltend eine fermentierbare Kohlenstoff-haltige Verbindung und eine Stickstoff-haltige Verbindung in Gegenwart von Mikroorganismen, insbesondere bei einem pH-Wert im Bereich von 3,0 bis 11, unter Bildung eines Verfahrensprodukts der Fermentation enthaltend Aminobenzoat-Anionen (H₂NC₅H₄COO⁻) und/oder Aminobenzoesäure (H₂NC₅H₄COOH bzw. H₃N⁺C₆H₄COO⁻);
(B)
   (I) Kristallisieren von Aminobenzoesäure während des Fermentierens durch Einstellen eines pH-Werts in der Fermentation im Bereich von 3,0 bis 4,7, bevorzugt 3,2 bis 3,7, besonders bevorzugt 3,4 bis 3,6, ganz besonders bevorzugt 3,5, gefolgt von Abtrennen der in der Kristallisation ausgefallenen Aminobenzoesäure durch Filtration, Sedimentation oder Zentrifugation unter Erhalt der wässrigen Mutterlauge enthaltend gelöste Aminobenzoesäure,
   (II) ***(1)*** *Partielles* Kristallisieren von Aminobenzoesäure während des Fermentierens gefolgt von Abtrennen der in der partiellen Kristallisation ausgefallenen Aminobenzoesäure durch Filtration, Sedimentation oder Zentrifugation unter Erhalt einer Lösung, und **(2)** Kristallisieren von Aminobenzoesäure aus der Lösung durch Einstellen eines pH-Werts im Bereich von 3,0 bis 4,7, bevorzugt 3,2 bis 3,7, besonders bevorzugt 3,4 bis 3,6, ganz besonders bevorzugt 3,5, gefolgt von Abtrennen der in der Kristallisation ausgefallenen Aminobenzoesäure durch Filtration, Sedimentation oder Zentrifugation unter Erhalt der wässrigen Mutterlauge enthaltend gelöste Aminobenzoesäure,
      oder
   (III) Kristallisieren von Aminobenzoesäure nach dem Fermentieren durch Einstellen eines pH-Werts im Verfahrensprodukt der Fermentation, optional (und bevorzugt) nachdem dieses von Mikroorganismen befreit wurde, im Bereich von 3,0 bis 4,7, bevorzugt 3,2 bis 3,7, besonders bevorzugt 3,4 bis 3,6, ganz besonders bevorzugt 3,5, gefolgt von Abtrennen der in der Kristallisation ausgefallenen Aminobenzoesäure durch Filtration, Sedimentation oder Zentrifugation unter Erhalt der wässrigen Mutterlauge enthaltend gelöste Aminobenzoesäure;
(C) Extraktion der wässrigen Mutterlauge enthaltend gelöste Aminobenzoesäure mit einem Alkanol mit 8 bis 12 Kohlenstoffatomen, bevorzugt 9 bis 11 Kohlenstoffatomen, unter Erhalt einer ersten alkoholischen Phase enthaltend Aminobenzoesäure und einer ersten wässrigen Phase;
(D) Rückextraktion von Aminobenzoesäure aus der ersten alkoholischen Phase mit (I) einer wässrigen Basenlösung oder (II) einer wässrigen Säurelösung unter Erhalt einer zweiten wässrigen Phase enthaltend (I) Anionen oder (II) Kationen der Aminobenzoesäure und einer zweiten alkoholischen Phase; und
(E) Kristallisieren von Aminobenzoesäure aus der zweiten wässrigen Phase
   (I) durch Führen der zweiten wässrigen Phase in die bei einem pH-Wert im Bereich von 3,0 bis 4,7, bevorzugt 3,2 bis 3,7, besonders bevorzugt 3,4 bis 3,6, ganz besonders bevorzugt 3,5, durchgeführte Kristallisation aus (B)(I), (B)(II)(2) oder (B)(III),
      und/oder (bevorzugt oder)
   (II) durch Einstellen eines pH-Werts im Bereich von 3,0 bis 4,7, bevorzugt 3,2 bis 3,7, besonders bevorzugt 3,4 bis 3,6, ganz besonders bevorzugt 3,5, in einem von (B)(I), (B)(II)(2) und (B)(III) verschiedenen Schritt, gefolgt von Abtrennen der hierbei ausgefallenen Aminobenzoesäure durch Filtration, Sedimentation oder Zentrifugation (wobei eine weitere wässrige Mutterlauge zurückbleibt, welche insbesondere entsorgt wird).

Vollkommen überraschend wurde gefunden, dass die Verwendung von C₈-C₁₂-Alkanolen, insbesondere von C₉-C₁₁-Alkanolen als Extraktionsmittel (Schritt (C)) in Verbindung mit einer basischen Rückextraktion (Schritt (D)) einen guten Kompromiss zwischen der Anforderung einer möglichst vollständigen Extraktion der Aminobenzoesäure aus der Mutterlauge (um eine möglichst hohe Ausbeute zu erzielen) auf der einen Seite und der Anforderung, eine möglichst reine extrahierte Mutterlauge zu erhalten (um die Abwasseraufbereitung bzw. -entsorgung zu vereinfachen) auf der anderen Seite darstellt.

Alle *pH-Werte* beziehen sich im Rahmen der vorliegenden Erfindung auf die Temperatur, bei welcher der entsprechende Schritt (z. B. Schritt (B) oder (E.II)) durchgeführt wird und können mit einer Glaselektrode einfach gemessen werden. Die Schritte Schritt (B) und (E.II)) werden insbesondere bei Umgebungstemperatur durchgeführt.

Es folgt zunächst eine Kurzzusammenfassung verschiedener möglicher **Ausführungsformen der Erfindung:**
In einer **ersten Ausführungsform** der Erfindung, die mit allen anderen Ausführungsformen kombiniert werden kann, wird als Alkanol ein primäres Alkanol eingesetzt.

In einer **zweiten Ausführungsform** der Erfindung, die eine besondere Ausgestaltung der ersten Ausführungsform ist, wird als primäres Alkanol 1-Decanol eingesetzt.

In einer **dritten Ausführungsform** der Erfindung, die mit allen anderen Ausführungsformen kombiniert werden kann, wird in der Variante (D)(I) als wässrige Basenlösung Natronlauge, Kalilauge, eine Natrium- oder Kaliumhydrogencarbonat-Lösung, eine Natrium- oder Kaliumcarbonat-Lösung oder eine Mischung von zwei oder mehr der vorgenannten Verbindungen eingesetzt, und in der Variante (D)(II) wird als wässrige Säurelösung Salzsäure, Schwefelsäure, Phosphorsäure oder eine Mischung von zwei oder mehr der vorgenannten Verbindungen eingesetzt.

In **einer vierten Ausführungsform** der Erfindung, die mit allen anderen Ausführungsformen kombiniert werden kann, wird in der Rückextraktion in (D) ein molares Verhältnis von Hydroxidionen (in der Variante (D)(I)) bzw. Hydroniumionen (in der Variante (D)(**II**)) zu Aminobenzoesäure von 1,0 bis 5,0, bevorzugt 1,0 bis 2,0 und besonders bevorzugt 1,0 bis 1,5 eingehalten.

In einer fünften **Ausführungsform** der Erfindung, die mit allen anderen Ausführungsformen kombiniert werden kann, erfolgt das Einstellen des pH-Werts in (B) und/oder in (E.II) durch Zugabe einer Säure ausgewählt aus Salzsäure, Schwefelsäure oder Phosphorsäure oder durch Zugabe einer Base ausgewählt aus wässrigem Ammoniak, gasförmigen Ammoniak, Natronlauge, Kalilauge, einer Natrium- oder Kaliumhydrogencarbonat-Lösung oder einer Natrium- oder Kaliumcarbonat-Lösung. In den Varianten (B)(II) und insbesondere (B)(III) kann auch das (dann Aminobenzoat-Anionen enthaltende) Verfahrensprodukt der Fermentation zur pH-Wert-Einstellung in E(II) verwendet werden.

In einer **sechsten Ausführungsform** der Erfindung, die mit allen anderen Ausführungsformen kombiniert werden kann, wird die Extraktion in (C) bei einer Temperatur von 20 °C bis 90 °C, bevorzugt 25 °C bis 70 °C, besonders bevorzugt 30 °C bis 50 °C durchgeführt.

In einer **siebten Ausführungsform** der Erfindung, die mit allen anderen Ausführungsformen kombiniert werden kann, wird die Rückextraktion in (D) bei einer Temperatur von 20 °C bis 90 °C, bevorzugt 25 °C bis 70 °C, besonders bevorzugt 30 °C bis 50 °C durchgeführt.

In einer **achten Ausführungsform** der Erfindung, die mit allen anderen Ausführungsformen kombiniert werden kann, ist
- die fermentierbare Kohlenstoff-haltige Verbindung ausgewählt aus Stärkehydrolysat, Zuckerrohrsaft, Zuckerrübensaft, Hydrolysate aus lignocellulosehaltigen Rohstoffen oder einer Mischung von zwei oder mehr der vorgenannten Kohlenstoff-haltigen Verbindung,
   und
- die Stickstoff-haltige Verbindung ausgewählt aus Ammoniakgas, Ammoniakwasser, (mindestens) einem Ammoniumsalz, Sojaprotein, Harnstoff oder einer Mischungen von zwei oder mehr der vorgenannten Stickstoff-haltigen Verbindungen.

In einer **neunten Ausführungsform** der Erfindung, die mit allen anderen Ausführungsformen kombiniert werden kann, sind die Mikroorganismen ausgewählt aus *Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii* oder *Saccharomyces cerevisiae.*

In einer **zehnten Ausführungsform** der Erfindung, die mit allen anderen Ausführungsformen kombiniert werden kann, wird die zweite alkoholische Phase, optional nach einer Aufreinigung, in die Extraktion von (C) zurückgeführt.

In einer **elften Ausführungsform** der Erfindung, die mit allen anderen Ausführungsformen kombiniert werden kann, wird
(F) die erste wässrigen Phase durch Destillation in eine dritte wässrige Phase und eine dritte alkoholische Phase getrennt, wobei die dritte wässrige Phase als Abwasser entsorgt wird und die dritte alkoholische Phase in die Extraktion von (C) zurückgeführt wird.

In einer **zwölften Ausführungsform** der Erfindung, die mit allen anderen Ausführungsformen kombiniert werden kann, wird (E.II) durchgeführt.

In einer **dreizehnten Ausführungsform** der Erfindung, die mit allen anderen Ausführungsformen kombiniert werden kann, wird ortho-Aminobenzoesäure hergestellt.

In einer **vierzehnten Ausführungsform** der Erfindung, die mit allen anderen Ausführungsformen kombiniert werden kann, wird para-Aminobenzoesäure hergestellt.

Die zuvor kurz geschilderten Ausführungsformen und weitere mögliche Ausgestaltungen der Erfindung werden im Folgenden näher erläutert. Sämtliche zuvor beschriebenen Ausführungsformen und die weiteren im Folgenden beschriebenen Ausgestaltungen der Erfindung sind, sofern sich aus dem Kontext für den Fachmann nicht eindeutig das Gegenteil ergibt oder nicht ausdrücklich etwas anderes gesagt wird, beliebig unter- und miteinander kombinierbar.

### BEREITSTELLEN DER, INSBESONDERE VON MIKROORGANISMEN BEFREITEN (= GEKLÄRTEN), FERMENTATIONSBRÜHE

**Schritt (A)** des erfindungsgemäßen Verfahrens betrifft die *Fermentation einer fermentierbaren Kohlenstoff-haltigen Verbindung und einer Stickstoff-haltigen Verbindung in Gegenwart von Mikroorganismen unter Bildung von Aminobenzoat-Anionen (H₂NC₆H₄COO⁻; und*/*oder Aminobenzoesäure (H₂NC₆H₄COOH bzw. H₃N⁺C₆H₄COO⁻).* Die Fermentation wird in einem dafür vorgesehenen Reaktionsapparat, dem Fermentationsreaktor, durchgeführt. Das im Fermentationsreaktor vorliegende Reaktionsgemisch wird als *Fermentationsbrühe* bezeichnet. Die nach erfolgter Fermentation vorliegende Fermentationsbrühe wird im Rahmen der vorliegenden Erfindung auch als *Verfahrensprodukt der Fermentation* bezeichnet.

Die Fermentation in Schritt (A) wird bevorzugt so durchgeführt, dass der pH-Wert in der Fermentationsbrühe im Bereich von 3,0 bis 11, bevorzugt 6,0 bis 8,0, liegt. Erforderlichenfalls kann der pH-Wert durch Zugabe von wässrigem oder gasförmigen Ammoniak, wässrigem Kaliumhydroxid oder wässrigem Natriumhydroxid (bei zu niedrigen pH-Werten) bzw. durch Zugabe einer wässrigen Säure, insbesondere von Salzsäure, Schwefelsäure oder Salpetersäure (bei zu hohen pH-Werten) reguliert werden. Für unterschiedliche Mikroorganismen können unterschiedliche pH-Bereiche innerhalb der genannten Bereiche besonders optimal sein; dies wird im Folgenden näher ausgeführt.

Bevorzugte Mikroorganismen für die Durchführung von Schritt (I) sind **Prokaryoten** (wie insbesondere Bakterien) oder **Eukaryoten** (wie insbesondere Hefen). Besonders bevorzugt sind dabei Mikroorganismen wie *Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii* oder *Saccharomyces cerevisiae,* wobei der alleinige Einsatz von *Corynebacterium glutamicum, insbesondere Corynebacterium glutamicum ATCC 13032,* besonders bevorzugt ist.

Der in der Fermentation einzuhaltende pH-Wert richtet sich wie bereits erwähnt nach dem eingesetzten Mikroorganismus. Mikroorganismen wie *Corynebacterium glutamicum, Pseudomonas putida* oder *Escherichia coli* werden bevorzugt bei "neutralen bis basischen pH-Werten" (d. h. insbesondere bei einem pH-Wert im Bereich von 6,0 bis 11, bevorzugt 6,0 bis 8,0) kultiviert. Mikroorganismen wie *Saccharomyces cerevisiae* werden hingegen bevorzugt im sauren Milieu kultiviert (d. h. insbesondere bei einem pH-Wert im Bereich von 3,0 bis < 6,0, bevorzugt 4,0 bis < 6,0). Abhängig vom konkret innerhalb des Bereiches von 3,0 bis 11 vorliegendem pH-Wert liegt die Aminobenzoesäure in der Fermentationsbrühe als Anion (H₂NC₆H₄COO⁻) oder in der elektroneutralen Form (H₂NC₆H₄COOH bzw. H₃N⁺C₆H₄COO⁻) vor (die Bildung von Kationen H₃N⁺C₆H₄COOH bei pH-Werten im untersten Teil des genannten Bereiches, also bei pH 3,0 oder wenig darüber, ist nicht ganz auszuschließen, macht aber allenfalls einen untergeordneten Anteil der insgesamt vorhandenen Aminobenzoesäure aus):
Bei pH-Werten im Bereich insbesondere von 4,7 oder weniger, bevorzugt von 3,7 oder weniger, besonders bevorzugt von 3,6 oder weniger, liegt die Aminobenzoesäure überwiegend bis vollständig in der elektroneutralen Form vor *und kristallisiert daher während der Fermentation spontan aus,* sodass ein separater Kristallisationsschritt verzichtbar ist und die kristallisierte Aminobenzoesäure direkt aus der Fermentationsbrühe isoliert werden kann **(Variante (I) von Schritt (B)).** Bei pH-Werten insbesondere von > 4,7, bevorzugt von 6,0 oder mehr, besonders bevorzugt von 8,0 oder mehr, liegt die Aminobenzoesäure überwiegend bis vollständig als Anion vor, sodass ein separater Kristallisationsschritt - **Variante (III) von Schritt (B);** siehe unten für Details - durchgeführt wird. Es ist ebenfalls denkbar - **Variante (II) von Schritt (B)** - die Fermentation bei solchen pH-Werten durchzuführen, dass zwar bereits Aminobenzoesäure während der Fermentation ausfällt, der Anteil der als Anion gelösten Aminobenzoesäure aber noch durch eine zusätzliche, *nach* der Fermentation durchgeführte pH-Wert-Anpassung weiter verringert werden kann. **Variante (III)** - die Durchführung der Fermentation bei solchen pH-Werten, dass Aminobenzoesäure während der Fermentation überwiegend bis vollständig in anionischer Form gelöst bleibt und die Kristallisation in einem separaten Schritt durchgeführt wird - ist von den drei Varianten die am stärksten bevorzugte.

In einer bevorzugten Ausgestaltung der Erfindung werden **Prokaryoten, insbesondere Bakterien als Mikroorganismen** eingesetzt. Dabei wird insbesondere auf die Patentanmeldungen WO 2015/124686 A1 und WO 2015/124687 A1 verwiesen, in denen Fermentationsprozesse unter Einsatz von Bakterien beschrieben sind (siehe beispielsweise WO 2015/124687 A1, (i) Seite 15, Zeile 8 bis Seite 16, Zeile 30, (ii) Beispiel 1 (Seite 29, Zeile 4 bis 26), (iii) Beispiel 3 (vor allem Seite 34, Zeile 10 bis 18), (iv) Beispiel 4 (vor allem Seite 55, Zeile 9 bis 31) und die das Anion der Aminobenzoesäure (Aminobenzoat-Anion) als unmittelbares Produkt der Fermentation liefern, also für die **Variante (III)** geeignet sind. Insbesondere kommen solche Bakterien zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

In einer anderen bevorzugten Ausgestaltung der Erfindung werden **Eukaryoten, insbesondere Hefen als Mikroorganismen** eingesetzt. Dabei wird insbesondere auf die internationale Anmeldung WO 2017/102853 A1 verwiesen. Insbesondere kommen Hefezellen zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht. Hefen werden bevorzugt im sauren Milieu kultiviert und eignen sich daher für die **Varianten (I) und (II).**

Um derartige Prokaryoten oder derartige Eukaryoten zu erhalten, stehen grundsätzlich zwei Wege zur Verfügung, die in bevorzugter Ausgestaltung auch kombiniert werden können:
(i) Die enzymatischen Reaktionen im Aminobenzoesäure-Stoffwechselweg der prokaryotischen oder eukaryotischen Zelle können so erhöht werden, dass Aminobenzoesäure schneller produziert als verbraucht wird.
(ii) Die Folgereaktionen, durch welche Aminobenzoesäure in weitere Metaboliten oder Produkte (z. B. Tryptophan) überführt wird, können reduziert bzw. ausgeschaltet werden, sodass sogar die Geschwindigkeit der Aminobenzoesäure-Bildung in Wildtyp-Stämmen ausreichend ist, um zu einer Anreicherung von Aminobenzoesäure in der Zelle zu führen.

Verfahren zur Gewinnung von prokaryotischen oder eukaryotischen Organismen mit den zuvor genannten Eigenschaften sind im Stand der Technik bekannt. Geeignete Prokaryoten oder Eukaryotenkönnen beispielsweise durch Screening nach Mutanten, welche Aminobenzoesäure in das umgebende Medium abgeben, identifiziert werden. Die zielgerichtete Modifikation von Schlüsselenzymen mittels gentechnischer Verfahren ist jedoch bevorzugt. Mit üblichen gentechnischen Methoden können Genexpression und Enzymaktivität verstärkt, verringert oder sogar ganz unterbunden werden. Es resultieren rekombinante Stämme. Für das besonders bevorzugte ortho-Isomer wird eine bevorzugte Ausführungsform im Folgenden beschrieben; eine Übertragung auf die anderen Isomere liegt im Rahmen üblichen fachmännischen Könnens:
Besonders bevorzugt enthalten die Prokaryoten oder Eukaryoten, die in der Lage sind, eine fermentierbare Kohlenstoff-haltige Verbindung in Gegenwart einer Stickstoff-haltigen Verbindung zu Aminobenzoesäure umzusetzen, eine Modifizierung der Anthranilat-Phosphoribosyltransferase-Aktivität, welche besagte Enzymaktivität herabsetzt. Durch diese Modifizierung wird die Umwandlung von ortho-Aminobenzoat zu N-(5-Phospho-D-Ribosyl)-Anthranilat reduziert oder komplett unterbunden. Hierdurch wird eine Anreicherung von Aminobenzoesäure in der Zelle bewirkt. Die Bezeichnung "Anthranilat-Phosphoribosyltransferase-Aktivität" bezieht sich dabei auf eine Enzymaktivität, durch welche die Umsetzung von ortho-Aminobenzoat zu N-(5-Phospho-D-Ribosyl)-Anthranilat katalysiert wird.

In Hefen wird die Anthranilat-Phosphoribosyltransferase-Aktivität durch das native Gen TRP4 (YDR354W) genetisch kodiert. In dem Bakterium *Corynebacterium glutamicum* wird die Anthranilat-Phosphoribosyltransferase-Aktivität durch das *trpD* Gen (cg3361, Cgl3032, NCgl2929) kodiert. Im Falle von *Pseudomonas putida* erfolgt die Kodierung über das *trpD* Gen (PP_0421) innerhalb des *trpDC* Operons.

Die beschriebene Herabsetzung der Anthranilat-Phosphoribosyltransferase-Aktivität kann prinzipiell auf drei Wegen erreicht werden:
(i) Die Regulierung der Expression des Gens für die Anthranilat-Phosphoribosyltransferase-Aktivität kann so modifiziert werden, dass die Transkription des Gens oder anschließende Translation verringert oder unterbunden wird.
(ii) Die Nukleinsäuresequenz des Gens für Anthranilat-Phosphoribosyltransferase-Aktivität kann so modifiziert werden, dass das Enzym, welches durch das modifizierte Gen kodiert wird, eine geringere spezifische Aktivität aufweist.
(iii) Das native Gen für Anthranilat-Phosphoribosyltransferase-Aktivität kann durch ein anderes Gen, das von einem verschiedenen Organismus stammt, ersetzt und ein Enzym mit einer spezifischen Anthranilat-Phosphoribosyltransferase-Aktivität, die geringer ist als die der zuvor erwähnten nativen Gene (z.B. TRP4, *trpD* oder *trpDC),* kodiert werden.

Aminobenzoesäure kommt in drei *isomeren Formen (ortho-, meta-* und para-Aminobenzoesäure) vor. Grundsätzlich kann das erfindungsgemäße Verfahren auf alle drei Isomere, entweder in isomerenreiner Form oder als Mischungen verschiedener Isomere, angewandt werden. Bevorzugt ist jedoch die Herstellung von *ortho-*Aminobenzoesäure oder para-Aminobenzoesäure, insbesondere in isomerenreiner Form. Besonders bevorzugt ist die Herstellung von ortho-Aminobenzoesäure, insbesondere in isomerenreiner Form. *"Isomerenrein"* bedeutet in diesem Zusammenhang in der Terminologie der vorliegenden Erfindung, dass der Stoffmengenanteil des gewünschten Aminobenzoesäure-Isomers, bezogen auf alle vorhandenen Aminobenzoesäure-Isomere, mindestens 99,0 mol-%, bevorzugt mindestens 99,9 mol-%, besonders bevorzugt 100 mol-% beträgt. Wie in der Fachwelt bekannt ist, kann die Bildung des gewünschten Isomers enzymatisch gesteuert werden. So kann etwa im Shikimatweg Chorismat enzymatisch zu Anthranilat (= Anion der ortho-Aminobenzoesäure) umgesetzt werden.

Alternativ gibt es auch Enzym-katalysierte Reaktionen vom Chorismat zu *para-*Aminobenzoat (= Anion der para-Aminobenzoesäure).

Unabhängig davon, welcher Mikroorganismus eingesetzt und welches Isomer angestrebt wird, umfasst die Fermentationsbrühe zu Beginn der Fermentation in Schritt (A) rekombinante Zellen des eingesetzten Mikroorganismus und mindestens eine fermentierbare Kohlenstoff-haltige Verbindung (sowie mindestens eine Stickstoff-haltige Verbindung als Stickstoffquelle). Bevorzugt enthält die Fermentationsbrühe darüber hinaus noch weitere Bestandteile ausgewählt aus der Gruppe bestehend aus Puffersystemen, anorganischen Nährstoffen, Aminosäuren, Vitaminen und weiteren organischen Verbindungen welche für das Wachstum bzw. den Erhaltungsstoffwechsel der rekombinanten Zellen benötigt werden. Die Fermentationsbrühe ist wasserbasiert. Nach Einsetzen des Fermentationsprozesses umfasst die Fermentationsbrühe auch Aminobenzoesäure, das angestrebte Fermentationsprodukt.

Wie bereits erwähnt, wird unter einer fermentierbaren Kohlenstoff-haltigen Verbindung im Sinne der vorliegenden Erfindung jede organische Verbindung oder Mischung organischer Verbindungen verstanden, die von den rekombinanten Zellen des eingesetzten Mikroorganismus verwendet werden kann, um Aminobenzoesäure zu produzieren. Die Produktion von Aminobenzoesäure kann dabei in Gegenwart oder Abwesenheit von Sauerstoff stattfinden.

Bevorzugt sind dabei solche fermentierbaren Kohlenstoff-haltigen Verbindungen, die zusätzlich als Energie- und Kohlenstoffquelle für das Wachstum der rekombinanten Zellen des eingesetzten Mikroorganismus dienen können. Besonders geeignet sind Stärkehydrolysat, Zuckerrohrsaft, Zuckerrübensaft und/oder Hydrolysate aus lignocellulosehaltigen Rohstoffen. Als Stickstoffquelle wird vorzugsweise Ammoniakgas, Ammoniakwasser, (mindestens) ein Ammoniumsalz, Sojaprotein und/oder Harnstoff eingesetzt.

In einer Ausführungsform der Erfindung wird Schritt (A) kontinuierlich durchgeführt, d. h. dass die Edukte dem Fermentationsreaktor kontinuierlich zugeführt und das Produkt dem Fermentationsreaktor kontinuierlich entnommen wird. Das dem Fermentationsreaktor kontinuierlich entnommene Produkt ist dabei im einfachsten Fall die Fermentationsbrühe inklusive der darin vorhandenen Mikroorganismen. Es ist jedoch auch denkbar, durch Anwendung bekannter Trennverfahren (insbesondere Filtration) die Mikroorganismen im Fermentationsreaktor zurückzuhalten und eine *geklärte* Fermentationsbrühe aus diesem zu entnehmen. Eine solche Klärung der Fermentationsbrühe kann selbstverständlich auch außerhalb des Fermentationsreaktors vorgenommen werden, insbesondere durch Filtration, Zentrifugation oder Sedimentation. In der Variante (III) ist die Klärung der Fermentationsbrühe vor der Kristallisation in Schritt (B) bevorzugt (siehe weiter unten für Details).

In einer anderen Ausführungsform der Erfindung wird Schritt (A) in einer diskontinuierlichen Verfahrensführung (sog. "Batch-Fahrweise") in *Fermentationszyklen* durchgeführt. Dabei umfasst ein Fermentationszyklus bevorzugt die initiale Vorlage oder Zugabe von Mikroorganismen in ein Nährmedium, die Vorlage und/oder Zugabe von Nährstoffen, den Aufbau von Mikroorganismen, die Bildung des gewünschten Produktes, also der Aminobenzoesäure, sowie die vollständige oder teilweise Entleerung des Reaktors nach Abschluss der Fermentation. In einer Variante der diskontinuierlichen Fahrweise (sog. "Fed-Batch-Fahrweise") werden die Edukte dem Fermentationsreaktor so lange (kontinuierlich oder diskontinuierlich [d. h. portionsweise]) zugeführt, wie es das Reaktorvolumen erlaubt, ohne dass Produkte - gegebenenfalls ausgenommen gasförmige Bestandteile, die über eine Anbindung des Fermentationsreaktor an ein Abgassystem ausgetragen werden - dem Fermentationsreaktor entnommen werden. Die Reaktion wird nach Zugabe der maximal möglichen Menge an Edukten unterbrochen und das Produktgemisch dem Fermentationsreaktor entnommen. Auch bei diskontinuierlicher Verfahrensführung ist die Klärung der Fermentationsbrühe, insbesondere durch Filtration, Zentrifugation oder Sedimentation, vor der Kristallisation in Schritt (B) gemäß Variante (III) bevorzugt.

Unabhängig von der genauen Fahrweise umfasst der Fermentationsreaktor bevorzugt Einrichtungen zur Messung wichtiger Prozessparameter wie Temperatur, pH-Wert, Konzentration an Substrat und Produkt, Gehalt an gelöstem Sauerstoff, Zelldichte der Fermentationsbrühe. Insbesondere bevorzugt umfasst der Fermentationsreaktor Einrichtungen zur Anpassung von mindestens einem (bevorzugt von allen) der vorgenannten Prozessparameter.

Geeignete Fermentationsreaktoren sind Rührkessel, Membranreaktoren, Kolbenstromreaktoren *("plug flow reactors")* oder Schlaufenreaktoren. Besonders bevorzugt sowohl für aerobe als auch für anaerobe Fermentationen sind Rührkesselreaktoren und Schlaufenreaktoren (bevorzugt Airliftreaktoren, in denen die Zirkulation der Flüssigkeit im Reaktor durch Begasung erreicht wird).

### KRISTALLISATION DER AMINOBENZOESÄURE AUS DER, INSBESONDERE GEKLÄRTEN, FERMENTATIONSBRÜHE

In **Schritt (B)** des erfindungsgemäßen Verfahrens wird die Aminobenzoesäure *auskristallisiert.*

Dies geschieht in der **Variante (I)** (ausschließlich) im Fermentationsreaktor selbst, also noch während der Fermentation gemäß Schritt (A). Die ausgefallene Aminobenzoesäure wird nach Beendigung der Fermentation durch Filtration, Sedimentation oder Zentrifugation abgetrennt, wobei diese im Gemisch mit den Mikroorganismen anfällt. Zur Trennung beider kann beispielsweise so vorgegangen werden, dass man die Aminobenzoesäure *in möglichst konzentrierter Form* selektiv löst (z. B. mit konzentrierter Basenlösung oder mit einem organischen Lösungsmittel) und dann aus dieser Lösung erforderlichenfalls wieder abscheidet (durch erneute Kristallisation bzw. Eindampfen). Als organisches Lösungsmittel eignet sich zum Beispiel Anilin. Dies ist auch insbesondere dann vorteilhaft, wenn die gewonnene Aminobenzoesäure zu Anilin decarboxyliert werden soll, weil dann kein systemfremdes Lösungsmittel eingeführt wird. Außerdem kann die Lösung von Aminobenzoesäure in Anilin direkt decarboxyliert werden, wobei das Anilin sogar eine katalytische Wirkung entfaltet. Bei der Aufarbeitung der Verfahrensproduktes der Fermentation nach Variante (II) kann genauso vorgegangen werden wie hier für die Variante (I) beschrieben.

In den **Varianten (II) und (III)** wird neben dem Fermentationsreaktor ein zusätzlicher für Kristallisationen geeigneter technischer Apparat, in der Fachwelt als *Kristallisator* bezeichnet, eingesetzt. Die Kristallisation in einem solchen Kristallisator kann für die Varianten (II) und (III) in gleicher Weise durchgeführt werden und wird daher im Folgenden für beide Varianten gemeinsam beschrieben:
Geeignete Kristallisatoren sind beispielsweise Rührkessel oder Zwangsumlaufkristallisatoren wie solche vom "Typ Oslo". Im Kristallisator wird der pH-Wert auf einen Wert im Bereich von 3,0 bis 4,7, bevorzugt 3,2 bis 3,7, besonders bevorzugt 3,4 bis 3,6 und ganz besonders bevorzugt 3,5 eingestellt. Dies geschieht vorzugsweise durch Zugabe einer Säure ausgewählt aus Salzsäure, Schwefelsäure oder Phosphorsäure. Durch diese pH-Wert-Einstellung werden die Aminobenzoesäure-Anionen (H₂NC₆H₄COO⁻) überwiegend bis vollständig in die elektroneutrale Form ((H₂NC₆H₄COOH oder H₃N⁺C₆H₄COO⁻) überführt und kristallisieren aus. Diese Art der Kristallisation wird auch als Reaktivkristallisation bezeichnet. Die kristallisierte Aminobenzoesäure kann durch Filtration, Sedimentation oder Zentrifugation isoliert werden, wobei die *wässrige Mutterlauge enthaltend gelöste Aminobenzoesäure* zurückbleibt.

Es hat sich bewährt, die Fermentationsbrühe und die Säure dem Kristallisator über räumlich (möglichst weit) getrennte Zuführeinrichtungen zuzuführen. Hierdurch wird erreicht, dass die Reaktanden möglichst gut mit dem Reaktorinhalt vermischt werden, bevor es zur Säure-Base-Reaktion kommt. Als Zuführeinrichtungen kommen bspw. Rohrleitungen, bevorzugt mit Absperrventilen, in Betracht. In einer Ausführungsform sind die Zuführeinrichtung für die Fermentationsbrühe und die Zuführeinrichtung für die Säure an gegenüberliegenden Stellen der Reaktorwandung (im Wesentlichen) rechtwinklig zu dieser angeordnet. In einer anderen Ausführungsform sind die Zuführeinrichtung für die Fermentationsbrühe und die Zuführeinrichtung für die Säure (im Wesentlichen) parallel zur Reaktorwandung angeordnet, wobei sich die Zuführeinrichtungen gegenüberliegen und möglichst nahe, insbesondere unmittelbar, an der Reaktorwand befinden.

Es ist möglich, den in den Varianten (II) und (III) des Schritts (B) eingesetzten Kristallisator durch geeignete Einbauten in Kammern zu unterteilen. Durch Wahl von Rührergeometrie und Rührerbetriebsweise kann die Strömungsrichtung eingestellt werden. Es ist ebenfalls möglich, den Kristallisator mit einem externen Umpumpkreislauf zu versehen, wobei dann einer der beiden Reaktanden - Fermentationsbrühe oder Säure - in den Umpumpkreislauf und der andere direkt in den Kristallisator gegeben wird. Wird ein Kristallisator mit Sichter und Umpumpkreislauf betrieben, wird der Umpumpkreislauf am Sichterboden zur Aufwirbelung oder seitlich des Sichters eingesetzt.

Die Kristallisation im Kristallisator kann kontinuierlich oder diskontinuierlich durchgeführt werden. Die kontinuierliche Durchführung ist bevorzugt. Unabhängig von der Fahrweise (kontinuierlich oder diskontinuierlich) werden die genauen Betriebsparameter (u. a.) durch die gewünschte Kristallgröße bestimmt, welche durch die Verweilzeit/Reaktionszeit und den Übersättigungsgrad eingestellt werden kann (große Kristallgrößen werden durch lange Verweilzeiten/lange Reaktionszeiten und geringe Übersättigungsgrade begünstigt).

Die Kristallisation wird bevorzugt in Anwesenheit von Impfkristallen durchgeführt:
Vorzugsweise geht man bei einer diskontinuierlich durchgeführten Kristallisation so vor, dass man die Fermentationsbrühe zunächst im Kristallisator vorlegt und auf eine definierte Temperatur (bevorzugt 5 °C bis 40 °C, zum Beispiel 20 °C) temperiert. Liegt der pH-Wert der Fermentationsbrühe signifikant oberhalb der Löslichkeitsgrenze von Aminobenzoesäure bei der gewählten Temperatur, so säuert man die Fermentationsbrühe vor der eigentlichen Säurezugabe zunächst leicht an (bevorzugt mit einer der bereits oben erwähnten Säuren Salzsäure, Schwefelsäure oder Phosphorsäure, besonders bevorzugt mit Salzsäure, insbesondere mit 37%iger Salzsäure), und zwar auf einen pH-Wert, der der Löslichkeitsgrenze von Aminobenzoesäure bei der gewählten Temperatur entspricht oder zumindest nahe bei dieser liegt (bevorzugt pH 5,2 bis 5,8, insbesondere pH 5,5). Dieses leichte Ansäuern kann rasch erfolgen. Dann gibt man Impfkristalle des gewünschten Polymorphs der Aminobenzoesäure zu, und zwar vorzugsweise Polymorph (Form) I. Dieses Polymorph hat eine vergleichsweise geringe Löslichkeit und begünstigt daher die möglichst vollständige Gewinnung der Aminobenzoesäure. Die Menge der zugegebenen Impfkristalle beträgt vorzugsweise etwa 0,1 % bis 1 % der in der Fermentationsbrühe gelösten Aminobenzoesäure. Auf diese Weise wird eine Suspension an Impfkristallen erhalten (siehe auch WO 2017/085170 A1). Anschließend wird der pH-Wert durch Zugabe von Säure (im Fall des vorangehenden leichten Ansäuerns der gleichen Säure wie dort verwendet) auf 3,0 bis 4,7, bevorzugt 3,2 bis 3,7, besonders bevorzugt 3,4 bis 3,6, ganz besonders bevorzugt 3,5, eingestellt. Dabei wird die Säure vorzugsweise langsam zugegeben; zum Beispiel bei 1 kg vorgelegter Fermentationsbrühe und Verwendung 37%iger Salzsäure innerhalb von 1 h. Nach beendeter Säurezugabe wird noch eine gewisse Zeit nachgerührt, insbesondere für den gleichen Zeitraum, den die Zugabe der Säure nach Zugabe der Impfkristalle in Anspruch nahm. Die ausgefallene Aminobenzoesäure wird anschließend durch Filtration, Sedimentation oder Zentrifugation abgetrennt (vorzugsweise durch Vakuum-Filtration) und vorzugsweise mehrfach (insbesondere zweifach) mit einer wässrigen sauren Waschflüssigkeit (insbesondere der gleichen Säure, die zur Fällung eingesetzt wurde) eines pH-Werts von 3,0 bis 4,7, bevorzugt 3,2 bis 3,7, besonders bevorzugt 3,4 bis 3,6, ganz besonders bevorzugt 3,5, gewaschen.

Bei einer kontinuierlich durchgeführten Kristallisation muss man Impfkristalle im Allgemeinen nur bei der Inbetriebnahme des kontinuierlichen Prozesses gezielt zugeben, da sich weitere Impfkristalle später *in situ* von selbst bilden (sog. Sekundärkeimbildung). Die Bereitstellung der zur Inbetriebnahme benötigten Suspension an Impfkristallen kann dabei so erfolgen, wie zuvor für die diskontinuierliche Kristallisation beschrieben. Die Aufarbeitung (Abtrennung und Wäsche der kristallisierten Aminobenzoesäure) kann ebenfalls erfolgen wie zuvor beschrieben.

Die Varianten (II) und (III) unterscheiden sich bei der Kristallisation im Kristallisator nur in einem Punkt, nämlich darin, dass das mit Säure zu behandelnde Ausgangsmaterial im Fall der Variante (II) eine *Lösung* ist (nämlich die nach Abtrennung der bereits während der Fermentation ausgefallenen Aminobenzoesäure erhaltene Lösung; *bei dieser Abtrennung werden suspendierte Mikroorganismen ebenfalls mit abgetrennt)* und im Fall der Variante (III) eine *Suspension* der Mikroorganismen im Verfahrensprodukt der Fermentation. Im Fall der Variante (III) ist es daher bevorzugt, das Verfahrensprodukt der Fermentation vor Durchführung der Kristallisation von den darin suspendierten Mikroorganismen durch Filtration, Zentrifugation oder Sedimentation zu befreien (zu *klären).* Geschieht dies nicht, fällt bei der Abtrennung der in der Kristallisation ausgefallenen Aminobenzoesäure dieselbe im Gemisch mit Mikroorganismen an. Zur Trennung beider kann so vorgegangen werden, wie weiter oben für die Variante (I) beschrieben (selektives Lösen der Aminobenzoesäure und erforderlichenfalls erneutes Abscheiden der Aminobenzoesäure aus der erhaltenen Lösung). Da dies jedoch aufwändig ist und Ausbeuteverluste nicht ausgeschlossen werden können, ist die Abtrennung der Mikroorganismen *vor* der Kristallisation bevorzugt.

Neben der bevorzugt durchgeführten Klärung kann die Fermentationsbrühe aus Schritt (A) weiteren Vorbehandlungsschritten unterzogen werden, ehe sie dem Schritt (B) zugeführt wird. Hier ist insbesondere eine *Entfärbung* der (insbesondere geklärten) Fermentationsbrühe zu nennen. Eine solche Entfärbung wird bevorzugt so durchgeführt, dass die Fermentationsbrühe bzw. von Mikroorganismen befreite Fermentationsbrühe über eine Säule mit fester Packung geleitet wird, um Farbstoffe mittels Adsorption zu entfernen. Als mögliche feste Phase kann z. B. Kieselgur oder eine Ionenaustauscherpackung verwendet werden. Eine solche Entfärbung wird bevorzugt dann durchgeführt, wenn in der Fermentationsbrühe solche farbigen Substanzen vorhanden sind, die die nachfolgende Kristallisation in Schritt (B) stören könnten. In der Variante (II) kann die nach Abtrennung der bereits in der Fermentation kristallisierten Aminobenzoesäure erhaltene Lösung vor der Kristallisation in (B)(III) ebenfalls einer solchen Entfärbung unterzogen werden.

### EXTRAKTION DER WÄSSRIGEN MUTTERLAUGE ENTHALTEND GELÖSTE AMINOBENZOESÄURE MIT EINEM C₈-C₁₂, INSBESONDERE C₉-C₁₁-ALKANOL

In **Schritt** (C) des erfindungsgemäßen Verfahrens wird die in Schritt (B) anfallende Mutterlauge enthaltend gelöste Aminobenzoesäure mit einem Alkanol mit 8 bis 12, bevorzugt 9 bis 11 Kohlenstoffatomen *extrahiert.* Dabei werden nach Phasentrennung eine erste alkoholische Phase enthaltend Aminobenzoesäure und eine erste wässrige Phase erhalten. Bevorzugt werden primäre Alkanole, besonders bevorzugt 1-Decanol als Extraktionsmittel eingesetzt. Die Extraktion kann bei Temperaturen von 20 °C bis 90 °C, bevorzugt 25 °C bis 70°C, besonders bevorzugt 30 °C bis 50 °C durchgeführt werden, insbesondere auch bei Umgebungstemperatur. Als Apparate für die Durchführung der Extraktion eignen sich beispielsweise sog. *Mixer Settler oder* Extraktionskolonnen mit einer theoretischen Trennstufenzahl von bevorzugt 3 bis 10 Stufen, besonders bevorzugt 3 bis 7 Stufen, ganz besonders bevorzugt 4 bis 6 Stufen. Das massenbezogene Phasenverhältnis der organischen zur wässrigen Phasen beträgt bevorzugt 0,20 bis 1,0, besonders bevorzugt 0,20 bis 0,50, ganz besonders bevorzugt 0,35 bis 0,50.

Die Extraktion löst nicht nur Aminobenzoesäure aus der Mutterlauge, sondern auch organische Verunreinigungen. Dies hat den Vorteil, dass die extrahierte Mutterlauge (= die *erste wässrige Phase)* verhältnismäßig rein vorliegt, was deren weitere Reinigung (siehe *Schritt (F)* weiter unten) erleichtert.

### RÜCKEXTRAKTION VON AMINOBENZOESÄURE AUS DER ERSTEN ALKOHOLISCHEN PHASE

In **Schritt (D)** des erfindungsgemäßen Verfahrens wird die in Schritt (B) anfallende erste alkoholische Phase mit einer wässrigen Basenlösung **(I)** oder Säurelösung (II) *extrahiert,* um die Aminobenzoesäure wieder aus dieser alkoholischen Phase *zurückzugewinnen* (sog. *Rückextraktion).* Bei der Behandlung mit (I) Base oder (II) Säure wird die Wasserlöslichkeit der Aminobenzoesäure durch Überführung in eine insgesamt negativ (durch Deprotonierung mit einer Base) oder positiv (durch Protonierung mit einer Säure) geladene Form so gesteigert, dass die erhaltenen Anionen bzw. Kationen überwiegend bis vollständig in die wässrige Phase übertreten. Hierbei werden daher nach Phasentrennung eine zweite wässrige Phase enthaltend im Fall (I) Anionen (H₂NC₆H₄COO⁻) bzw. im Fall (Il) Kationen (H₃N⁺C₆H₄COOH) der Aminobenzoesäure und eine zweite alkoholische Phase erhalten. Da in der Fermentation organische Verunreinigungen anfallen, die vergleichsweise polar sind (siehe die Beispiele) und insbesondere diese vergleichsweise polaren Verunreinigungen in diesem Schritt zumindest in Anteilen in die zweite wässrige Phase übergehen, ist es bevorzugt, Maßnahmen zum Ausschleusen solcher Verunreinigungen zu treffen (siehe hierzu die Beschreibung des Schritts (E) weiter unten). Genau wie Schritt (B) kann auch diese Extraktion bei Temperaturen von 20 °C bis 90 °C, bevorzugt 25 °C bis 70 °C, besonders bevorzugt 30 °C bis 50 °C, insbesondere auch bei Umgebungstemperatur, durchgeführt werden.

Als wässrige Basenlösung für Schritt (D)(I) wird bevorzugt Natronlauge, Kalilauge, eine Natrium- oder Kaliumhydrogencarbonat-Lösung, eine Natrium- oder Kaliumcarbonat-Lösung oder (weniger bevorzugt) eine Mischung von zwei oder mehr der vorgenannten Verbindungen eingesetzt. Natron- und Kalilauge sind besonders bevorzugt. Unabhängig von der eingesetzten Base ist es bevorzugt, ein molares Verhältnis von Hydroxidionen zu Aminobenzoesäure von 1,0 bis 5,0, bevorzugt 1,0 bis 2,0 und besonders bevorzugt 1,0 bis 1,5 einzuhalten. Als wässrige Säurelösung für Schritt (D)(II) wird bevorzugt Salzsäure, Schwefelsäure, Phosphorsäure oder (weniger bevorzugt) eine Mischung von zwei oder mehr der vorgenannten Verbindungen eingesetzt. Bevorzugt sind Salzsäure oder Schwefelsäure. Unabhängig von der eingesetzten Säure ist es bevorzugt, ein molares Verhältnis von Hydroniumionen (H₃O⁺-Ionen) zu Aminobenzoesäure von 1,0 bis 5,0, bevorzugt 1,0 bis 2,0 und besonders bevorzugt 1,0 bis 1,5 einzuhalten.

Abhängig von der gewählten Verfahrensweise ist die zweite wässrige Phase also basisch (pH insbesondere 7,0 oder größer, bevorzugt 7,0 bis 14, besonders bevorzugt 7,0 bis 13; *(D)(I))* oder sauer (pH insbesondere 2,0 oder kleiner, bevorzugt 0,0 bis 2,0, besonders bevorzugt 0,0 bis 1,0; *(D)(II))).*

Für diese Rückextraktion eignen sich die gleichen Apparate wie zuvor für Schritt (C) beschrieben. Das massenbezogene Phasenverhältnis der organischen zur wässrigen Phasen beträgt bevorzugt 0,20 bis 1,0, besonders bevorzugt 0,20 bis 0,50, ganz besonders bevorzugt 0,20 bis 0,40.

Die nach Phasentrennung erhaltene zweite alkoholische Phase wird vorzugsweise, optional nach einer Aufreinigung zur Entfernung von Verunreinigungen, die in der Phasentrennung in diese Phase gelangt sind, in die Extraktion von Schritt (C) zurückgeführt und dort als Bestandteil (ggf. auch alleiniger Bestandteil) des als Extraktionsmittel einzusetzenden C₈-C₁₂-, insbesondere C₉-C₁₁-Alkanols eingesetzt. Eine optional durchgeführte Aufreinigung umfasst insbesondere eine Destillation.

### KRISTALLISATION VON AMINOBENZOESÄURE AUS DER ZWEITEN WÄSSRIGEN PHASE

Im folgenden **Schritt (E)** des erfindungsgemäßen Verfahrens wird die in der zweiten wässrigen Phase gelöste Aminobenzoesäure *auskristallisiert.* Hierzu muss der pH-Wert der zweiten wässrigen Phase auf einen Wert im Bereich von 3,0 bis 4,7, bevorzugt 3,2 bis 3,7, besonders bevorzugt 3,4 bis 3,6, ganz besonders bevorzugt 3,5, eingestellt werden, was durch pH-Verringerung (bei basischer Rückextraktion gemäß (D)(I)) bzw. pH-Erhöhung (bei saurer Rückextraktion gemäß (D)(II)) geschieht.

Im einfachsten Fall kann dies erfolgen, indem - (E)(I) - die zweite wässrige Phase in die Kristallisation aus Schritt (B) zurückgeführt wird, nämlich in eine Kristallisation gemäß **(B)(I), (B)(II)(2)** oder **(B)(III):**
Wird die zweite wässrige Phase zur Kristallisation *in die Fermentation* geführt (also in den Schritt (B)(I), muss der nach erfolgter Vermischung von Fermentationsbrühe und zweiter wässriger Phase resultierende pH-Wert in einem Bereich sein, der die Kristallisation der Aminobenzoesäure zur Folge hat (siehe die Beschreibung der Schritte (A) und (B) weiter oben für Details). Ob überhaupt und wenn ja in welchem Umfang zu diesem Zweck neben der zweiten wässrigen Phase weitere Säure oder Base zugegeben werden muss, entscheidet sich abhängig von den Umständen des Einzelfalls und ist für den Fachmann unmittelbar ersichtlich. Bevorzugt wird bei Rückführung des zweiten wässrigen Phase in die Kristallisation gemäß (B)(I) die Rückextraktion basisch durchgeführt (also gemäß (D)(I)). Die Dosierung der dann basischen zweiten wässrigen Phase in die - in dieser Variante saure - Fermentationsbrühe wird so vorgenommen, dass der resultierende pH-Wert im angestrebten Bereich von 3,0 bis 4,7, bevorzugt 3,2 bis 3,7, besonders bevorzugt 3,4 bis 3,6, ganz besonders bevorzugt 3,5, liegt.
Wird die zweite wässrige Phase zur Kristallisation *in einen vom Fermentationsreaktor verschiedenen Kristallisator geführt* (also in den Schritt **(B)(II)(2)** oder **(B)(III)),** muss der nach erfolgter Vermischung der *Lösung aus (B)(II)(1)* bzw. des *Verfahrensprodukts der Fermentation* und zweiter wässriger Phase resultierende pH-Wert im einem Bereich sein, der die Kristallisation von Aminobenzoesäure ermöglicht, also im Bereich von 3,0 bis 4,7, bevorzugt 3,2 bis 3,7, besonders bevorzugt 3,4 bis 3,6, ganz besonders bevorzugt 3,5. In den Varianten (B)(II) und (B)(III) ist der pH-Wert an dieser Stelle (d. h. in der Lösung aus (B)(1) bzw. im Verfahrensprodukt der Fermentation) *verhältnismäßig hoch,* sodass Aminobenzoat-Anionen vorliegen. Der pH-Wert der Lösung aus (B)(1) bzw. des Verfahrensprodukts der Fermentation muss also verringert werden. Bei Durchführung der Rückextraktion im Sauren gemäß (D)(II) wird der hierzu erforderliche Bedarf an Säure teilweise durch die zweite wässrige Phase selbst gedeckt. Bei Durchführung der Rückextraktion im Basischen gemäß (D)(I) erhöht sich der Säurebedarf durch Zugabe der zweiten wässrigen Phase entsprechend.
Bei Durchführung des Schritts (E) gemäß (E)**(I)** kristallisiert also die Aminobenzoesäure *aus der zweiten wässrigen Phase* gemeinsam mit der Aminobenzoesäure *aus der Fermentationsbrühe* aus und wird auch gemeinsam mit dieser weiter verarbeitet wie beschrieben (siehe WEITERE VERARBEITUNG DER AMINOBENZOESÄURE). In der zweiten wässrigen Phase gelöste organische Verunreinigungen werden in dieser Variante bevorzugt über Purge-Ströme ausgeschleust.

Es ist jedoch auch möglich - **(E)(II)** - die zweite wässrige Phase in einem von (B)(I), (B)(II)(2) und (B)(III) verschiedenen Schritt in einen (vergleichsweise kleinen und vom in Schritt (B)(II)(2) und (B)(III) eingesetzten Kristallisator verschiedenen) Kristallisator zu führen und dort Aminobenzoesäure auszukristallisieren:
Hierzu muss der pH-Wert auf einen Wert im Bereich von 3,0 bis 4,7, bevorzugt 3,2 bis 3,7, besonders bevorzugt 3,4 bis 3,6, ganz besonders bevorzugt 3,5, eingestellt werden. Die Einstellung des pH-Werts erfolgt bevorzugt
**(i)** - (bei Durchführung der Rückextraktion gemäß **(D)(I))** - durch Zugabe einer Säure ausgewählt aus Salzsäure, Schwefelsäure oder Phosphorsäure bzw.
**(ii)** - bei Durchführung der Rückextraktion gemäß **(D)(II)** - durch Zugabe einer Base ausgewählt aus wässrigem Ammoniak, gasförmigen Ammoniak, Natronlauge, Kalilauge, einer Natrium- oder Kaliumhydrogencarbonat-Lösung oder einer Natrium- oder Kaliumcarbonat-Lösung oder auch durch Zugabe eines Teil des Verfahrensproduktes der Fermentation. Letzteres setzt natürlich voraus, dass der pH-Wert des Verfahrensprodukts der Fermentation signifikant über dem pH-Wert der zweiten wässrigen Phase liegt und auch hoch genug ist, um die erhaltene Mischung auf einen pH-Wert im Bereich von 3,0 bis 4,7, bevorzugt 3,2 bis 3,7, besonders bevorzugt 3,4 bis 3,6, ganz besonders bevorzugt 3,5, zu bringen. Daher ist die Verwendung eines Teils des Verfahrensproduktes der Fermentation zur pH-Wert-Erhöhung der zweiten wässrigen Phase bei saurer Rückextraktion auf die Varianten (B)(II) und (B)(III) beschränkt und insbesondere für die Variante (B)(III) geeignet.

Nach Phasentrennung durch Filtration, Sedimentation oder Zentrifugation verbleibt in der Ausführungsform gemäß (E)(II) eine weitere wässrige Mutterlauge. Diese weitere wässrige Mutterlauge enthält nun die in Schritt (C) aus der in Schritt (B) anfallenden Mutterlauge extrahierten organischen Verunreinigungen zumindest zu einem beträchtlichen Anteil. Da die weitere wässrige Mutterlauge aus Schritt (D) in deutlich geringerer Menge anfällt (basierend auf den Phasenverhältnissen der beiden vorgelagerten Extraktionsschritten) als die erste wässrige Phase (die organischen Verunreinigungen also aufkonzentriert werden), liegt nun mit der weiteren wässrigen Mutterlauge eine vergleichsweise kleine wässrige Phase vor, die das Wertprodukt Aminobenzoesäure allenfalls noch in vernachlässigbar geringer Konzentration enthält, jedoch an organischen Verunreinigungen vergleichsweise reich ist und daher entsorgt werden kann. Auf diese Weise werden Nebenkomponenten auf einfache Weise aus dem Prozess ausgeschleust, sodass sich diese nicht so stark anreichern können. Das Anfallen der weiteren wässrigen Mutterlauge in vergleichsweise geringer Menge erleichtert weiterhin die Entsorgung (entweder durch Reinigung bspw. durch Ausfällen der organischen Verunreinigungen und anschließende Einspeisung in das Abwasser oder - wenn auch weniger bevorzugt - durch Verbrennung nach Eindampfung). Diese Ausführungsform bietet einen einfachen Auslass für organische Verunreinigungen aus dem Prozess und ist daher bevorzugt.

Es ist ebenfalls möglich, die zuvor genannten Ausführungsformen (E)(I) und (E)(II) derart miteinander zu verbinden, dass ein erster Teil der zweiten wässrigen Phase gemäß (E)(I) und ein zweiter Teil der zweiten wässrigen Phase gemäß (E)(II) weiter behandelt wird. Bevorzugt wird jedoch nur eine der Varianten (E)(I) oder (E)(II) durchgeführt. Dies gilt insbesondere dann, wenn die Fermentation bei sauren pH-Werten von 3,0 bis 4,7 durchgeführt wird und infolgedessen die Aminobenzoesäure gemäß der Variante (B)(I) während des Fermentierens spontan auskristallisiert. In diesem Fall ist es bevorzugt, die zweite wässrige Phase nur gemäß der Variante (E)(II) zu behandeln. Hierdurch wird verhindert, dass der Fermentation zusätzlich Säure zugegeben werden muss, um den pH-Wert im Bereich von 3,0 bis 4,7 zu halten.

### AUFARBEITUNG DER ERSTEN WÄSSRIGEN PHASE

Die in Schritt (C) anfallende erste wässrige Phase wird vorzugsweise in einem **Schritt (F)** *weiter aufgearbeitet.* Da diese erste wässrige Phase im erfindungsgemäßen Verfahren vergleichsweise rein anfällt, genügt hierfür eine einfache Destillation zur Abtrennung der (geringen) Anteile in der ersten wässrigen Phase gelösten Alkanols. Hierbei wird eine dritte alkoholische Phase abdestilliert, welche in die Extraktion von Schritt (C) zurückgeführt und dort als Bestandteil des als Extraktionsmittel einzusetzenden C₈-C₁₂-, insbesondere C₉-C₁₁-Alkanols eingesetzt wird. Bei der Destillation bleibt eine dritte wässrige Phase zurück, die als Abwasser entsorgt wird. Da im erfindungsgemäßen Verfahren die dritte wässrige Phase organische Verunreinigungen allenfalls in vergleichsweise geringen Anteilen enthält, kann sie im Allgemeinen (und wird es bevorzugt auch) direkt einer Kläranlage, insbesondere auch einer biologischen Kläranlage, zugeführt werden.

### WEITERE VERARBEITUNG DER AMINOBENZOESÄURE

Die in Schritt (B) und/oder in Schritt (E.II) gewonnene Aminobenzoesäure kann optional weiter gereinigt werden. Bei Durchführung von Schritt (E.II) geschieht dies bevorzugt nach Vereinigung mit der in Schritt (B) gewonnenen Aminobenzoesäure. Eine solche Reinigung ist an sich aus dem Stand der Technik bekannt (siehe vor allem WO 2015/124687 A1 und insbesondere auf WO 2015/124687 A1, Seite 18, Zeile 4 bis Seite 18, Zeile 6) und erfolgt bevorzugt durch eine oder mehrere Wäschen mit wässrigen Waschmedien, bevorzugt Wasser. Um Ausbeuteverluste zu vermeiden, wird der pH-Wert des wässrigen Waschmediums vorzugsweise auf einen Wert im Bereich von 3,0 bis < 4,0, bevorzugt auf einen Wert von 3,5 bis 3,7, eingestellt.

Die gegebenenfalls gereinigte Aminobenzoesäure kann optional in einem **Schritt (G)** *einer Umsetzung zu einem Aminobenzoesäure-Folgeprodukt* zugeführt werden, d. h. zu einem Produkt, das durch eine weitere chemische Umsetzung der Aminobenzoesäure erhalten wird. Bei Durchführung von Schritt (E.II) geschieht auch dies bevorzugt nach Vereinigung mit der in Schritt (B) gewonnenen Aminobenzoesäure. Ausgewählte weitere Umsetzungen der erhaltenen Aminobenzoesäure sind:
(I) Decarboxylierung der Aminobenzoesäure zu Anilin;
(II) Decarboxylierung der Aminobenzoesäure zu Anilin, gefolgt von säurekatalysierter Reaktion des Anilins mit Formaldehyd unter Bildung von Di- und Polyaminen der Diphenylmethanreihe;
(III) Decarboxylierung der Aminobenzoesäure zu Anilin, gefolgt von säurekatalysierter Reaktion des Anilins mit Formaldehyd unter Bildung von Di- und Polyaminen der Diphenylmethanreihe, gefolgt von Umsetzung mit Phosgen unter Bildung von Di- und Polyisocyanaten der Diphenylmethanreihe;
(IV) Decarboxylierung der Aminobenzoesäure zu Anilin, gefolgt von Umsetzung des Anilins zu einer Azoverbindung;
(V) Umsetzung der Aminobenzoesäure zu einem Amid; und
(VI) Umsetzung der Aminobenzoesäure zu einem leitenden Polymeren wie bevorzugt Polyanthranilsäure.

Derartige Umsetzungen sind im Stand der Technik bekannt und bedürfen daher keiner detaillierten Beschreibung an dieser Stelle.

Nachfolgend wird die Erfindung noch anhand von Beispielen näher erläutert.

### Beispiele

### Analytik:

Für die Auswertung der Verteilung einer Komponenten zwischen zwei Phasen wurden beide Phasen mittels HPLC-MS vermessen (mit identischer Probenvorbereitung und Methode). Die Verteilungskoeffizienten wurden daraufhin als Verhältnis der Peakflächen bei gleicher Retentionszeit bestimmt.

### Beispiele 1 bis 9: Extraktion von Aminobenzoesäure aus einer Mutterlauge

Eine Fermentationsbrühe enthaltend Anionen der ortho-Aminobenzoesäure (aus **Schritt (A))** wurde durch Filtration geklärt und danach einer Kristallisation mit Salzsäure bei pH = 3,5±0,2 unterworfen **(Schritt (B), Variante (III)).** Die verbleibende Mutterlauge wurde mit verschiedenen Lösungsmitteln einstufig extrahiert **(Schritt (C)).** Die Ausgangskonzentration von ortho-Aminobenzoesäure in der Mutterlauge betrug 8,7 g/L. Es wurde ein Phasenverhältnis von organischer zu wässriger Phase von 0,5 eingesetzt. Die Extraktion wurde bei 30 °C durchgeführt. Zur Bewertung der Versuche wurden die **Verteilungskoeffizienten *K*** (= Verhältnis der ortho-Aminobenzoesäure [oAB-]Konzentration in g/L in der organischen Phase [ORG = erste alkoholische Phase] zur wässrigen Phase [AQ = erste wässrige Phase]), die **Löslichkeiten** des Extraktionsmittels in der wässrigen Phase nach der Extraktion (= erste wässrige Phase) sowie - in vier Fällen - der **Massenanteil an organischen Nebenkomponenten** (NK) in der organischen ExtraktPhase (= erste alkoholische Phase) bezogen auf die Gesamtheit der organischen Nebenkomponenten in beiden Phasen (= m(NK)_{ORG/}m(NK)_{GES}) ermittelt. Hierzu wurde eine Trennung per HPLC durchgeführt, wobei organische Verunreinigungen, die früher als *ortho-*Aminobenzoesäure eluieren, als *polare Nebenkomponenten* und organische Verunreinigungen, die später als ortho-Aminobenzoesäure eluieren, als *unpolare Nebenkomponenten* summarisch zusammengefasst wurden.

**Tabelle 1: Verteilungskoeffizient, Wasserlöslichkeit und Nebenkomponentenverteilung bei verschiedenen Extraktionsmitteln**

| **Bsp.** | **Extraktionsmittel** | ***K =* c(oAB)_{ORG}/ c(oAB)_{AQ}** | **Löslichkeit in Wasser bei RT [g/L]** | **m(NK)_{ORG}/m(NK)_{GES}** | |
|---|---|---|---|---|---|
| | | | | **Polare Nebenkomponenten** | **Unpolare Nebenkomponenten** |
| 1 | Kerosin^{[a]} | 0,0 | < 0,02 | n. b. | n. b. |
| 2 | Isododecan^{[a]} | 0,0 | Unlöslich | n. b. | n. b. |
| 3 | Methylcyclopentan^{[a]} | 0,0 | Unlöslich | n. b. | n. b. |
| 4 | Toluol^{[a]} | 1,29 | 0,5 | 0,00 | 0,29 |
| 5 | Oleylalkohol^{[a]} | 6,82 | Unlöslich | n. b. | n. b. |
| 6 | 1-Decanol^{[b]} | 9,01 | 0,04 | 0,09 | 0,65 |
| 7 | 1-Dodecanol^{[b]} | 8,56 | 0,004 | n. b. | n. b. |
| 8 | Butylacetat^{[a]} | 21,63 | ^{~}6 | 0,08 | 0,62 |
| 9 | 2-Butanon^{[a]} | 26,12 | ^{~}250 | 0,24 | 0,73 |

| | | | | | |
|---|---|---|---|---|---|
| [a] Nicht erfindungsgemäßes Extraktionsmittel [b] Erfindungsgemäßes Extraktionsmittel n. b. = nicht bestimmt | | | | | |

Die Extraktion von ortho-Aminobenzoesäure mit Kohlenwasserstoffen gelang nur schlecht oder gar nicht. Die Extraktion mit Oleylalkohol verlief zwar besser, aber noch nicht zufriedenstellend genug. Erst die Extraktion mit den erfindungsgemäßen Extraktionsmitteln 1-Decanol und 1-Dodecanol führt zu technisch zufriedenstellenden Ergebnissen. Die Extraktion der ortho-Aminobenzoesäure gelingt zwar mit sehr polaren Lösungsmitteln wie Butylacetat und 2-Butanon noch besser, führt jedoch zu einer hohen Konzentration von Lösungsmittel in der wässrigen Phase. In Prozessen mit fermentativ gewonnener Aminobenzoesäure ist dies aufgrund der im Vergleich zu chemischen Prozessen sehr großen Volumina der wässrigen Ströme nicht akzeptabel.

Es zeigt sich, dass mit 1-Decanol organische Verunreinigungen ähnlich effektiv wie im Fall der Verwendung polarerer Lösungsmittel extrahiert werden können. Dies ermöglicht die Reduktion des Anteils an organischen Verunreinigungen in der ersten wässrigen Phase und die Ausschleusung derartiger Verunreinigungen wie weiter oben beschrieben.

### Beispiele 10 und 11: Basische Rückextraktion (Schritt (D)(I)) von ortho-Aminobenzoesäure aus Lösungen in Alkanolen

Ausgehend von diesen Ergebnissen wurde die Rückextraktion mit einer wässrigen Basenlösung (hier Natronlauge) untersucht. Es wurde jeweils für 1-Decanol **(Beispiel 10)** und 1-Dodecanol **(Beispiel 11)** die Extraktion aus verschiedenen synthetisch angereicherten organischen Phasen durchgeführt. Die Extraktion wurde mit einem molaren Verhältnis von NaOH zu Aminobenzoesäure von 1,2 durchgeführt. Es wurden Lösungen mit einer Konzentration an Aminobenzoesäure von 15 g/L, 20 g/L und 25 g/L mit einem massenbezogenen Phasenverhältnis von Basenlösung zu organischer Phase von 0,5 bei 30 °C extrahiert. Dabei konnte eine über alle Versuche gemittelte Rückgewinnung von Aminobenzoesäure von 80 % für die einstufige Extraktion aus 1-Decanol-Lösungen und 85 % für die einstufige Extraktion aus 1-Dodecanol-Lösungen gefunden werden.

### Beispiele 12 und 13: Saure Rückextraktion (Schritt (D)(II)) von ortho-Aminobenzoesäure aus einer Lösung in 1-Decanol

Ausgehend von diesen Ergebnissen wurde ferner die Rückextraktion mit einer wässrigen Säurelösung (hier Salzsäure oder Schwefelsäure) untersucht. Lösungen von *ortho-*Aminobenzoesäure in 1-Decanol (mit 1,5 Massen-% ortho-Aminobenzoesäure) wurden jeweils mit wässriger Säurelösung mit einer Konzentration 1,0 mol/L mit einem massenbezogenen Phasenverhältnis von organischer Phase zu Säurelösung von 1,0 bei 30 °C extrahiert. Bei Verwendung von Salzsäure als Extraktionsmittel wurden bei einstufiger Extraktion 87,4% **(Beispiel 12a),** bei zweistufiger Extraktion 98,1% **(Beispiel 12b)** und bei dreistufiger Extraktion 99,5 % **(Beispiel 12c)** der *ortho-*Aminobenzoesäure zurückgewonnen. Bei Verwendung von Schwefelsäure als Extraktionsmittel wurden bei einstufiger Extraktion 90,3 % **(Beispiel 13a),** bei zweistufiger Extraktion 99,3 % **(Beispiel 13b)** und bei dreistufiger Extraktion 99,3 % **(Beispiel 13c)** der ortho-Aminobenzoesäure zurückgewonnen.

## Patentansprüche

1. Verfahren zur Gewinnung von Aminobenzoesäure aus einer wässrigen Mutterlauge enthaltend gelöste Aminobenzoesäure, welche bei einer Kristallisation von Aminobenzoesäure anfällt, wobei das Verfahren umfasst:
(A) Fermentieren eines Rohstoffes enthaltend eine fermentierbare Kohlenstoff-haltige Verbindung und eine Stickstoff-haltige Verbindung in Gegenwart von Mikroorganismen unter Bildung eines Verfahrensprodukts der Fermentation enthaltend Aminobenzoat-Anionen und/oder Aminobenzoesäure;
(B)
(I) Kristallisieren von Aminobenzoesäure während des Fermentierens durch Einstellen eines pH-Werts in der Fermentation im Bereich von 3,0 bis 4,7, gefolgt von Abtrennen der in der Kristallisation ausgefallenen Aminobenzoesäure durch Filtration, Sedimentation oder Zentrifugation unter Erhalt der wässrigen Mutterlauge enthaltend gelöste Aminobenzoesäure,
(II) (1) Partielles Kristallisieren von Aminobenzoesäure während des Fermentierens gefolgt von Abtrennen der in der partiellen Kristallisation ausgefallenen Aminobenzoesäure durch Filtration, Sedimentation oder Zentrifugation unter Erhalt einer Lösung, und (2) Kristallisieren von Aminobenzoesäure aus der Lösung durch Einstellen eines pH-Werts im Bereich von 3,0 bis 4,7, gefolgt von Abtrennen der in der Kristallisation ausgefallenen Aminobenzoesäure durch Filtration, Sedimentation oder Zentrifugation unter Erhalt der wässrigen Mutterlauge enthaltend gelöste Aminobenzoesäure,
oder
(III) Kristallisieren von Aminobenzoesäure nach dem Fermentieren durch Einstellen eines pH-Werts im Verfahrensprodukt der Fermentation, im Bereich von 3,0 bis 4,7, gefolgt von Abtrennen der in der Kristallisation ausgefallenen Aminobenzoesäure durch Filtration, Sedimentation oder Zentrifugation unter Erhalt der wässrigen Mutterlauge enthaltend gelöste Aminobenzoesäure;
(C) Extraktion der wässrigen Mutterlauge enthaltend gelöste Aminobenzoesäure mit einem Alkanol mit 8 bis 12 Kohlenstoffatomen, unter Erhalt einer ersten alkoholischen Phase enthaltend Aminobenzoesäure und einer ersten wässrigen Phase;
(D) Rückextraktion von Aminobenzoesäure aus der ersten alkoholischen Phase mit (I) einer wässrigen Basenlösung oder (II) einer wässrigen Säurelösung unter Erhalt
einer zweiten wässrigen Phase enthaltend (I) Anionen der Aminobenzoesäure oder (II) Kationen der Aminobenzoesäure
und
einer zweiten alkoholischen Phase;
und
(E) Kristallisieren von Aminobenzoesäure aus der zweiten wässrigen Phase
(I) durch Führen der zweiten wässrigen Phase in die bei einem pH-Wert im Bereich von 3,0 bis 4,7, durchgeführte Kristallisation aus (B)(I), (B)(II)(2) oder (B)(III),
und/oder
(II) durch Einstellen eines pH-Werts im Bereich von 3,0 bis 4,7, in einem von (B)(I), (B)(II)(2) und (B)(III) verschiedenen Schritt, gefolgt von Abtrennen der hierbei ausgefallenen Aminobenzoesäure durch Filtration, Sedimentation oder Zentrifugation.

2. Verfahren nach Anspruch 1, bei welchem als Alkanol ein primäres Alkanol eingesetzt wird.

3. Verfahren nach Anspruch 2, bei welchem als primäres Alkanol 1-Decanol eingesetzt wird.

4. Verfahren nach einem der vorstehenden Ansprüche, bei welchem
in (D) (I) als wässrige Basenlösung Natronlauge, Kalilauge, eine Natrium- oder Kaliumhydrogencarbonat-Lösung, eine Natrium- oder Kaliumcarbonat-Lösung oder eine Mischung von zwei oder mehr der vorgenannten Verbindungen und
in (D) (II) als wässrige Säurelösung Salzsäure, Schwefelsäure, Phosphorsäure oder eine Mischung von zwei oder mehr der vorgenannten Verbindungen eingesetzt wird.

5. Verfahren nach einem der vorstehenden Ansprüche, bei welchem in der Rückextraktion in (D) (I) ein molares Verhältnis von Hydroxidionen zu Aminobenzoesäure von 1,0 bis 5,0 und in D (II) ein molares Verhältnis von Hydroniumionen zu Aminobenzoesäure von 1,0 bis 5,0 eingehalten wird.

6. Verfahren nach einem der vorstehenden Ansprüche, bei welchem das Einstellen des pH-Werts in (B) und/oder in (E.II)
(i) durch Zugabe einer Säure ausgewählt aus Salzsäure, Schwefelsäure oder Phosphorsäure
oder
(ii) durch Zugabe einer Base ausgewählt aus wässrigem Ammoniak, gasförmigen Ammoniak, Natronlauge, Kalilauge, einer Natrium- oder Kaliumhydrogencarbonat-Lösung oder einer Natrium- oder Kaliumcarbonat-Lösung oder durch Zugabe des Verfahrensprodukts der Fermentation enthaltend Aminobenzoat-Anionen
erfolgt.

7. Verfahren nach einem der vorstehenden Ansprüche, bei welchem die Extraktion in (C) bei einer Temperatur von 20 °C bis 90 °C durchgeführt wird.

8. Verfahren nach einem der vorstehenden Ansprüche, bei welchem die Rückextraktion in (D) bei einer Temperatur von 20 °C bis 90 °C durchgeführt wird.

9. Verfahren nach einem der vorstehenden Ansprüche, bei welchem
• die fermentierbare Kohlenstoff-haltige Verbindung ausgewählt ist aus Stärkehydrolysat, Zuckerrohrsaft, Zuckerrübensaft, Hydrolysate aus lignocellulosehaltigen Rohstoffen oder einer Mischung von zwei oder mehr der vorgenannten Kohlenstoff-haltigen Verbindung,
und
• die Stickstoff-haltige Verbindung ausgewählt ist aus Ammoniakgas, Ammoniakwasser, (mindestens) einem Ammoniumsalz, Sojaprotein, Harnstoff oder einer Mischungen von zwei oder mehr der vorgenannten Stickstoff-haltigen Verbindungen.

10. Verfahren nach einem der vorstehenden Ansprüche, bei welchem die Mikroorganismen ausgewählt sind aus *Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii oder Saccharomyces cerevisiae.*

11. Verfahren nach einem der vorstehenden Ansprüche, bei welchem die zweite alkoholische Phase, optional nach einer Aufreinigung, in die Extraktion von (C) zurückgeführt wird.

12. Verfahren nach einem der vorstehenden Ansprüche, bei welchem
(F) die erste wässrigen Phase durch Destillation in eine dritte wässrige Phase und eine dritte alkoholische Phase getrennt wird, wobei die dritte wässrige Phase als Abwasser entsorgt wird und die dritte alkoholische Phase in die Extraktion von (C) zurückgeführt wird.

13. Verfahren nach einem der vorstehenden Ansprüche, umfassend (E.II).

14. Verfahren nach einem der vorstehenden Ansprüche, bei welchem *ortho-*Aminobenzoesäure oder para-Aminobenzoesäure hergestellt wird.

15. Verfahren nach einem der vorstehenden Ansprüche, bei welchem das Verfahrensprodukt der Fermentation vor der Kristallisation gemäß (B)(III) von Mikroorganismen befreit wird.

## Claims

1. Process for recovering aminobenzoic acid from an aqueous mother liquor containing dissolved aminobenzoic acid which is obtained in a crystallization of aminobenzoic acid, wherein the process comprises:
(A) fermenting a raw material containing a fermentable carbon-containing compound and a nitrogen-containing compound in the presence of microorganisms to form a fermentation process product containing aminobenzoate anions and/or aminobenzoic acid;
(B)
(I) crystallizing aminobenzoic acid during the fermentation by establishing a pH in the fermentation in the range from 3.0 to 4.7, followed by removal of the aminobenzoic acid precipitated in the crystallization by filtration, sedimentation or centrifugation to obtain the aqueous mother liquor containing dissolved aminobenzoic acid,
(II) (1) partially crystallizing aminobenzoic acid during the fermentation, followed by removal of the aminobenzoic acid precipitated in the partial crystallization by filtration, sedimentation or centrifugation to obtain a solution, and (2) crystallizing aminobenzoic acid out of the solution by establishing a pH in the range from 3.0 to 4.7, followed by removal of the aminobenzoic acid precipitated in the crystallization by filtration, sedimentation or centrifugation to obtain the aqueous mother liquor containing dissolved aminobenzoic acid,
or
(III) crystallizing aminobenzoic acid after the fermentation by establishing a pH in the fermentation process product in the range from 3.0 to 4.7, followed by removal of the aminobenzoic acid precipitated in the crystallization by filtration, sedimentation or centrifugation to obtain the aqueous mother liquor containing dissolved aminobenzoic acid;
(C) extracting the aqueous mother liquor containing dissolved aminobenzoic acid with an alkanol having 8 to 12 carbon atoms to obtain a first alcoholic phase containing aminobenzoic acid and a first aqueous phase;
(D) re-extracting aminobenzoic acid from the first alcoholic phase with (I) an aqueous base solution or (II) an aqueous acid solution to obtain
a second aqueous phase containing (I) anions of aminobenzoic acid or (II) cations of aminobenzoic acid and
a second alcoholic phase;
and
(E) crystallizing aminobenzoic acid out of the second aqueous phase
(I) by guiding the second aqueous phase into the crystallization from (B)(I), (B)(II)(2) or (B)(III) that was conducted at a pH in the range from 3.0 to 4.7, and/or
(II) by establishing a pH in the range from 3.0 to 4.7, in a step other than (B)(I), (B)(II)(2) and (B)(III), followed by the removal of the aminobenzoic acid thus precipitated by filtration, sedimentation or centrifugation.

2. Process according to Claim 1, in which the alkanol used is a primary alkanol.

3. Process according to Claim 2, in which the primary alkanol used is 1-decanol.

4. Process according to any of the preceding claims, in which
the aqueous base solution used in (D)(I) is sodium hydroxide solution, potassium hydroxide solution, a sodium or potassium hydrogencarbonate solution, a sodium or potassium carbonate solution or a mixture of two or more of the aforementioned compounds and
the aqueous acid solution used in (D)(II) is hydrochloric acid, sulfuric acid, phosphoric acid or a mixture of two or more of the aforementioned compounds.

5. Process according to any of the preceding claims, in which, in the re-extraction in (D)(I), a molar ratio of hydroxide ions to aminobenzoic acid of 1.0 to 5.0 and, in (D)(II), a molar ratio of hydronium ions to aminobenzoic acid of 1.0 to 5.0 is observed.

6. Process according to any of the preceding claims, in which the pH is established in (B) and/or in (E.II)
(i) by adding an acid selected from hydrochloric acid, sulfuric acid or phosphoric acid
or
(ii) by adding a base selected from aqueous ammonia, gaseous ammonia, sodium hydroxide solution, potassium hydroxide solution, a sodium or potassium hydrogencarbonate solution or a sodium or potassium carbonate solution, or by adding the fermentation process product containing aminobenzoate anions.

7. Process according to any of the preceding claims, in which the extraction in (C) is performed at a temperature of 20°C to 90°C.

8. Process according to any of the preceding claims, in which the re-extraction in (D) is performed at a temperature of 20°C to 90°C.

9. Process according to any of the preceding claims, in which
• the fermentable carbon-containing compound is selected from starch hydrolysate, sugarcane juice, sugarbeet juice, hydrolysates of lignocellulosic raw materials or a mixture of two or more of the aforementioned carbon-containing compounds,
and
• the nitrogen-containing compound is selected from ammonia gas, aqueous ammonia, (at least) one ammonium salt, soya protein, urea or a mixture of two or more of the aforementioned nitrogen-containing compounds.

10. Process according to any of the preceding claims, in which the microorganisms are selected from Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii or Saccharomyces cerevisiae.

11. Process according to any of the preceding claims, in which the second alcoholic phase, optionally after a purification, is recycled into the extraction of (C).

12. Process according to any of the preceding claims, in which
(F) the first aqueous phase is separated by distillation into a third aqueous phase and a third alcoholic phase, where the third aqueous phase is disposed of as wastewater and the third alcoholic phase is recycled into the extraction of (C).

13. Process according to any of the preceding claims, comprising (E.II).

14. Process according to any of the preceding claims, in which ortho-aminobenzoic acid or para-aminobenzoic acid is produced.

15. Process according to any of the preceding claims, in which the fermentation process product is freed of microorganisms before the crystallization in (B)(III).

## Revendications

1. Procédé de récupération d'acide aminobenzoïque à partir d'une eau mère aqueuse contenant de l'acide aminobenzoïque dissous, laquelle est obtenue lors d'une cristallisation d'acide aminobenzoïque, le procédé comprenant :
(A) la fermentation d'une matière première contenant un composé fermentescible contenant du carbone et un composé contenant de l'azote en présence de microorganismes avec formation d'un produit de procédé de fermentation contenant des anions aminobenzoate et/ou de l'acide aminobenzoïque ;
(B)
(I) la cristallisation de l'acide aminobenzoïque pendant la fermentation en réglant une valeur de pH dans la fermentation dans la plage allant de 3,0 à 4,7, suivi d'une séparation de l'acide aminobenzoïque précipité lors de la cristallisation par filtration, sédimentation ou centrifugation, avec obtention de l'eau mère aqueuse contenant de l'acide aminobenzoïque dissous,
(II) (1) la cristallisation partielle d'acide aminobenzoïque pendant la fermentation, suivie d'une séparation de l'acide aminobenzoïque précipité lors de la cristallisation partielle par filtration, sédimentation ou centrifugation avec obtention d'une solution, et (2) la cristallisation d'acide aminobenzoïque à partir de la solution en réglant une valeur de pH dans la plage allant de 3,0 à 4,7, suivie d'une séparation de l'acide aminobenzoïque précipité lors de la cristallisation par filtration, sédimentation ou centrifugation, avec obtention de l'eau mère aqueuse contenant de l'acide aminobenzoïque dissous,
ou
(III) la cristallisation d'acide aminobenzoïque après la fermentation en réglant une valeur de pH dans le produit de procédé de fermentation dans la plage allant de 3,0 à 4,7, suivie d'une séparation de l'acide aminobenzoïque précipité lors de la cristallisation par filtration, sédimentation ou centrifugation, avec obtention de l'eau mère aqueuse contenant de l'acide aminobenzoïque dissous ;
(C) l'extraction de l'eau mère aqueuse contenant de l'acide aminobenzoïque dissous avec un alcanol comportant de 8 à 12 atomes de carbone, avec obtention d'une première phase alcoolique contenant de l'acide aminobenzoïque et d'une première phase aqueuse ;
(D) la réextraction d'acide aminobenzoïque à partir de la première phase alcoolique avec (I) une solution aqueuse basique ou (II) une solution aqueuse acide, avec obtention
d'une deuxième phase aqueuse contenant (I) des anions de l'acide aminobenzoïque ou (II) des cations de l'acide aminobenzoïque,
et
d'une deuxième phase alcoolique ;
et
(E) la cristallisation d'acide aminobenzoïque à partir de la deuxième phase aqueuse
(I) par introduction de la deuxième phase aqueuse dans la cristallisation réalisée à une valeur de pH dans la plage allant de 3,0 à 4,7 selon (B)(I), (B)(II)(2) ou (B)(III),
et/ou
(II) par réglage d'une valeur de pH dans la plage allant de 3,0 à 4,7, dans une étape différente de (B)(I), (B)(II)(2) et (B)(III), suivi d'une séparation de l'acide aminobenzoïque précipité à cette occasion par filtration, sédimentation ou centrifugation.

2. Procédé selon la revendication 1, dans lequel l'alcanol utilisé est un alcanol primaire.

3. Procédé selon la revendication 2, dans lequel le 1-décanol est utilisé comme alcanol primaire.

4. Procédé selon l'une des revendications précédentes, dans lequel
dans (D) (I), la solution aqueuse basique utilisée est une solution d'hydroxyde de sodium, une solution d'hydroxyde de potassium, une solution d'hydrogénocarbonate de sodium ou de potassium, une solution de carbonate de sodium ou de potassium, ou un mélange de deux ou plusieurs des composés précités, et
dans (D) (II), la solution aqueuse acide utilisée est de l'acide chlorhydrique, de l'acide sulfurique, de l'acide phosphorique ou un mélange de deux ou plusieurs des composés précités.

5. Procédé selon l'une des revendications précédentes, dans lequel, lors de la réextraction selon (D) (I), un rapport molaire d'ions hydroxyde à l'acide aminobenzoïque de 1,0 à 5,0 est respecté et, selon (D) (II), un rapport molaire d'ions hydronium à l'acide aminobenzoïque de 1,0 à 5,0 est respecté.

6. Procédé selon l'une des revendications précédentes, dans lequel le réglage de la valeur de pH dans (B) et/ou dans (E.II) est effectué
(i) par ajout d'un acide choisi parmi l'acide chlorhydrique, l'acide sulfurique ou l'acide phosphorique,
ou
(ii) par ajout d'une base choisie parmi l'ammoniac aqueux, l'ammoniac gazeux, une solution d'hydroxyde de sodium, une solution d'hydroxyde de potassium, une solution d'hydrogénocarbonate de sodium ou de potassium ou une solution de carbonate de sodium ou de potassium, ou par ajout du produit de procédé de fermentation contenant des anions aminobenzoate.

7. Procédé selon l'une des revendications précédentes, dans lequel l'extraction selon (C) est réalisée à une température de 20 °C à 90 °C.

8. Procédé selon l'une des revendications précédentes, dans lequel la réextraction selon (D) est réalisée à une température de 20 °C à 90 °C.

9. Procédé selon l'une des revendications précédentes, dans lequel
• la composition fermentescible contenant du carbone est choisie parmi un hydrolysat d'amidon, un jus de canne à sucre, un jus de betterave sucrière, des hydrolysats issus de matières premières contenant de la lignocellulose, ou un mélange de deux ou plusieurs des composés contenant du carbone précités,
et
• le composé contenant de l'azote est choisi parmi l'ammoniac gazeux, l'ammoniac aqueux, au moins un sel d'ammonium, une protéine de soja, l'urée ou un mélange de deux ou plusieurs des composés contenant de l'azote précités.

10. Procédé selon l'une des revendications précédentes, dans lequel les microorganismes sont choisis parmi Escherichia coli, Pseudomonas putida, Corynebacterium glutamicum, Ashbya gossypii, Pichia pastoris, Hansenula polymorpha, Kluyveromyces marxianus, Yarrowia lipolytica, Zygosaccharomyces bailii ou Saccharomyces cerevisiae.

11. Procédé selon l'une des revendications précédentes, dans lequel la deuxième phase alcoolique est recyclée dans l'extraction selon (C), éventuellement après une purification.

12. Procédé selon l'une des revendications précédentes, dans lequel
(F) la première phase aqueuse est séparée par distillation en une troisième phase aqueuse et une troisième phase alcoolique, la troisième phase aqueuse étant éliminée comme eau usée et la troisième phase alcoolique étant recyclée dans l'extraction selon (C).

13. Procédé selon l'une des revendications précédentes, comprenant (E.II).

14. Procédé selon l'une des revendications précédentes, dans lequel de l'acide ortho-aminobenzoïque ou de l'acide para-aminobenzoïque est produit.

15. Procédé selon l'une des revendications précédentes, dans lequel les microorganismes sont éliminés du produit de procédé de fermentation avant la cristallisation selon (B)(III).
